# FASCICULE DE BREVET EUROPEEN

(11) **EP 2 846 766 B1**
(45) Date de publication et mention de la délivrance du brevet: **25.05.2016**
(21) Numéro de dépôt: 13706182.6
(22) Date de dépôt: 30.01.2013
(51) Int. Cl.: A61K 9/00, A61K 9/51, A61K 49/18, A61K 49/00, C01F 7/02, A61K 9/50, C01G 15/00

(54) **NANOPARTICULES À LUMINESCENCE PERSISTANTE EXCITABLES IN SITU POUR L'IMAGERIE OPTIQUE IN VIVO, L'IMAGERIE MULTIMODALE OPTIQUE-IRM IN VIVO, ET LA THÉRANOSTIQUE**
IN-SITU-ERREGBARE NANOPARTIKEL MIT DAUERHAFTER LUMINESZENZ FÜR OPTISCHE IN-VIVO-BILDGEBUNG, MULTIMODALE OPTISCHE/MRT-BILDGEBUNG UND THERANOSTIK
PERSISTENT LUMINESCENCE NANOPARTICLES EXCITABLE IN SITU FOR IN VIVO OPTICAL IMAGING, IN VIVO MULTIMODAL OPTICAL/MRI IMAGING, AND THERANOSTICS

(30) Priorité: 30.01.2012 FR 1250846
(43) Date de publication de la demande: 18.03.2015
(73) Titulaire: Centre National de la Recherche Scientifique (CNRS), 75016 Paris (FR)
(72) Inventeur: MALDINEY, Thomas, F-75014 Paris (FR); RICHARD, Cyrille, F-78180 Montigny Le Bretonneux (FR); SCHERMAN, Daniel, F-75012 Paris (FR); GOURIER, Didier, F-75013 Paris (FR); VIANA, Bruno, F-91230 Montgeron (FR); BESSIERE, Aurélie, F-75018 Paris (FR)
(74) Mandataire: Regimbeau
(86) Numéro de dépôt international: PCT/EP2013/051727
(87) Numéro de publication internationale: WO 2013/113721

(56) Documents cités:
- WO-A1-2007/048856
- KIM J. ET AL.: "Ultrasmall c(RGDyK)-Coated Fe3O4 Nanoparticles and Their specific Targeting to integrin alphavbeta3-Rich Tumor Cells", ANGEW. CHEM., vol. 120, 1 janvier 2008 (2008-01-01), pages 8566-8569, XP002688653, cité dans la demande
- XIE, J. ET AL.: "Ultrasmall c(RGDyK)-Coated Fe3O4 Nanoparticles and Their specific Targeting to integrin alphavbeta3-Rich Tumor Cells", J. A. CHEM. SOC., vol. 130, 24 mai 2008 (2008-05-24), pages 7542-7543, XP002688654,
- DHAK, P. ET AL.: "Optical emission spectra of chromium doped nanocrystalline zinc gallate", JOURNAL OF APPLIED PHYSICS, vol. 106, 1 janvier 2009 (2009-01-01), - 1 janvier 2009 (2009-01-01), pages 063721-1-063721-6, XP012124019,
- BESSIÈRE A. ET AL.: "ZnGa2O4:Cr3+: a new red long-lasting phopshor with high brightness", OPTICS EXPRESS, vol. 19, 9 mai 2011 (2011-05-09), pages 10131-10137, XP002688656, cité dans la demande

## Description

L'invention a pour objet des méthodes d'imagerie optique dans lesquelles la luminescence persistante de nanoparticules est excitée *in vivo,* après injection à un organisme humain ou animal, puis est suivie en temps réel par émission d'un rayonnement prolongé situé dans le proche infra-rouge. La mise en oeuvre de nanoparticules ayant en outre des propriétés magnétiques permet une imagerie bimodale optique-imagerie par résonance magnétique (IRM). Par ailleurs, ces nanoparticules lorsqu'elles sont enrobées d'une couche de silice mésoporeuse ont une capacité de chargement et de relargage de molécules d'intérêt pouvant trouver des applications en théranostique.

La plupart des systèmes optiques utilisés pour le diagnostic disponibles sur le marché reposent sur des phénomènes de fluorescence qui nécessitent une excitation continue de la sonde. Cette excitation est cependant à l'origine d'un signal parasite des tissus de l'animal, l'auto-fluorescence, responsable d'une baisse significative de la sensibilité au moment de la détection.

La demande internationale WO2007/048856 et la demande FR 2 908 891 décrivent des nanoparticules à luminescence persistante et leur utilisation en tant qu'agent de diagnostic *in vivo.* L'excitation des nanoparticules s'effectue *in vitro* avant l'injection par irradiation sous une lampe UV. Une fois injectées à l'animal, ces nanoparticules peuvent être suivies plusieurs dizaines de minutes à travers le corps de l'animal, mais toute observation sur une durée supérieure à 60 minutes est rendue inopérante de par la durée de vie trop courte du signal de luminescence et l'impossibilité d'exciter les nanoparticules après injection, à travers le corps de l'animal.

Kim et al. (2004) ont décrit la préparation de nanoparticules de type ZnGa₂O₄:Cr³⁺ ainsi que leurs propriétés optiques de photoluminescence, mais ne rapportent aucune propriété de luminescence persistante. Des applications dans le domaine des télévisions à haute définition sont envisagées. Plus récemment, Bessière et al. (2011) ont démontré que ce même matériau, sous forme micrométrique, possédait des propriétés de luminescence persistante excitables dans l'UV à 254 nm. Pan et al. (2012) ont décrit les propriétés de luminescence persistante d'un gallogermanate de formule Zn₃Ga₂Ge₂O₁₀ :Cr³⁺, dont la taille des cristaux est comprise entre 2 et 5 µm, et proposent de les utiliser comme marquage de nuit, dans le domaine du stockage de l'énergie solaire et dans les méthodes de diagnostic décrites par Le Masne de Chermont et al. (2007) dans lesquelles les nanomatériaux sont excités avant leur injection à l'organisme animal.

La présente invention permet de lever une limitation essentielle de la première génération de nanoparticules à luminescence persistante en ce qu'elle peut être excitée *in vivo* à travers les tissus de l'animal. Bien que l'excitation *in vitro* sous UV soit encore possible, les nanoparticules utilisées dans les méthodes de d'imagerie optique de la présente invention ont la capacité d'être excitées dans le domaine des longueurs d'ondes visibles entre 550 et 800nm et plus particulièrement entre 550 et 650nm. Ces photons orange-rouges, relativement proches de la fenêtre de transparence des tissus, se révèlent assez pénétrants pour permettre une excitation de la luminescence persistante des nanoparticules *in vivo,* à travers le corps de l'animal. De cette manière, l'observation s'affranchit de toute contrainte de temps, et les nanoparticules peuvent être localisées tant que l'intégrité du cristal est conservée. De plus, il est dès lors possible de suivre leur cinétique de dégradation par imagerie optique non invasive.

Cette nouvelle génération de nanoparticules à luminescence persistante présente l'avantage d'un très fort rapport signal sur bruit et d'un contraste absolu de par l'absence d'autofluorescence. Cependant, les phénomènes de diffusion rencontrés en imagerie optique rendent parfois imprécise la localisation des nanoparticules au sein de l'organe observé *in vivo.* Ce problème technique est surmonté par l'apport d'une autre modalité, l'imagerie par résonance magnétique, qui permet alors de situer la sonde avec précision dans l'organe d'intérêt.

L'ajout d'un cation paramagnétique dans la structure de ces nanomatériaux permet d'apporter une modalité d'imagerie supplémentaire, l'IRM, sans affecter les propriétés initiales de luminescence persistante ni la capacité des nanoparticules à être excitées à travers les tissus de l'animal. Ce nanomatériau possède les mêmes propriétés optiques que celles décrites ci-dessus, ainsi que des propriétés magnétiques d'intérêt permettant son utilisation comme agent de contraste pour l'IRM (effet T2, contraste négatif).

L'invention offre également la possibilité d'encapsuler les nanoparticules à luminescence persistante dans une coquille de silice mésoporeuse pour permettre le chargement et relargage de molécules d'intérêt. En particulier, ce système multifonctionnel peut permettre de localiser, par le phénomène de luminescence persistante, les zones de relargage d'un principe actif ou d'une molécule cytotoxique, et ainsi de mieux contrôler l'administration d'un médicament par un suivi en temps réel de sa localisation dans l'organisme.

Ces nanoparticules et méthodes trouvent de nombreuses applications dans le domaine de l'imagerie médicale, du diagnostic et de la thérapie.

### RESUME

L'invention a pour objet une méthode d'imagerie optique *in vivo* d'un organisme humain ou animal comprenant les étapes suivantes :
a) Excitation de la luminescence persistante de nanoparticules par irradiation *in vivo* dans une longueur d'onde comprise entre 550 et 1000nm de tout ou partie de l'organisme humain ou animal, lesdites nanoparticules ayant été préalablement administrées à l'organisme humain ou animal et lesdites nanoparticules émettant des photons à des longueurs d'onde comprises entre 550 et 1000nm pendant au moins 0.01 seconde, après une excitation lumineuse à des longueurs d'onde comprises entre 550 et 1000nm et les dites nanoparticules comprenant un nanomatériau constitué d'une matrice choisie parmi les gallates, les aluminates, les indates, et leurs composés mixtes gallogermanates, gallo-aluminates, gallo-indates, les oxydes de gallium, les oxydes d'indium, les oxydes de magnésium, les oxysulfures zinc et de gallium, les oxysélénures de zinc et de gallium, les oxytellures de zinc et de gallium, ladite matrice étant dopée avec un métal de transition ou un lanthanide choisi parmi le chrome, l'europium, le cérium, le nickel, le fer, le cuivre et le cobalt; et
b) Détection *in vivo* des nanoparticules dans tout ou partie de l'organisme humain ou animal par mesure de la luminescence persistante des nanoparticules par imagerie optique.

De préférence, les nanoparticules comprennent le nanomatériau ZnGa₂₍₁₋ₓ₎Cr₂ₓO₄ avec x compris entre 0.001 et 0.0075. Plus préférentiellement, les nanoparticules comprennent le nanomatériau ZnGa_{1.995}Cr_{0.005}O₄.

De préférence, l'administration des nanoparticules est préalablement effectuée par voie intraveineuse, intraartérielle, intramusculaire, intrapéritonéale ou rétro-orbitale.

De préférence, les nanoparticules ont une taille comprise entre 1 et 10³ nm.

Dans des modes de réalisation préférés, les nanoparticules sont greffées en surface ou enrobées.

Dans un premier mode de réalisation, les nanoparticules sont greffées en surface avec un ligand. Dans un autre mode de réalisation, les nanoparticules sont encapsulées dans de la silice mésoporeuse permettant le chargement et le relargage de molécules d'intérêt.

L'invention a également pour objet une méthode d'imagerie bimodale *in vivo* d'un organisme humain ou animal comprenant les étapes suivantes :
a) Excitation de la luminescence persistante des nanoparticules par irradiation in vivo dans une longueur d'onde comprise entre 550 et 1000nm de tout ou partie de l'organisme humain ou animal, lesdites nanoparticules ayant été préalablement administrées à l'organisme humain ou animal et lesdites na nanoparticules émettant des photons à des longueurs d'onde comprises entre 550-1000 nm pendant au moins 0.01 seconde, après une excitation lumineuse à des longueurs d'onde comprises entre 550 et 1000nm et lesdites nanoparticules ayant des propriétés paramagnétiques et les dites nanoparticules comprenant un nanomatériau constitué d'une matrice parmi les gallates, les aluminates, les indates , les oxydes de gallium, les oxydes d'indium, les oxydes de magnésium, les gallogermanates, les aluminogallates, les oxysulfures zinc et de gallium, les oxysélénures de zinc et de gallium, les oxytellures de zinc et de gallium, ladite matrice étant dopée avec un métal de transition ou un lanthanide choisi parmi le chrome, l'europium, le cérium, le nickel, le fer, le cuivre, et le cobalt et avec au moins un élément paramagnétique choisi parmi Cr³⁺ ; Mn²⁺ ; Gd³⁺ ; Fe³⁺ et Ni³⁺.;
b) Détection *in vivo* des nanoparticules dans tout ou partie de l'organisme humain ou animal par mesure de la luminescence persistante des nanoparticules par imagerie optique ; et
c) Détection *in vivo* des nanoparticules dans tout ou partie de l'organisme humain ou animal par imagerie par résonance magnétique.

De préférence, les nanoparticules comprennent un nanomatériau ZnGa_{2(1-x-y)}Cr₂ₓGd₂yO₄ avec x compris entre 0.001 et 0.0075 et y compris entre 0.01 et 0.08. Plus préférentiellement, les nanoparticules comprennent le nanomatériau ZnGa_{1.955}Cr_{0.005}Gd_{0.04}O₄.

De préférence, l'administration des nanoparticules est préalablement effectuée par voie intraveineuse, intraartérielle, intramusculaire, intrapéritonéale ou rétro-orbitale.

De préférence, les nanoparticules ont une taille comprise entre 1 et 10³ nm.

Dans des modes de réalisation préférés, les nanoparticules sont greffées en surface ou enrobées.

Dans un premier mode de réalisation, les nanoparticules sont greffées en surface avec un ligand. Dans un autre mode de réalisation, les nanoparticules sont encapsulées dans de la silice mésoporeuse permettant le chargement et le relargage de molécules d'intérêt.

L'invention se rapporte à des nanoparticules comprenant le nanomatériau ZnGa_{2(1-x-y)}Cr₂ₓGd_{2y}O₄ avec x compris entre 0.001 et 0.0075 et y compris entre 0.01 et 0.08.

De préférence, les nanoparticules comprennent le nanomatériau ZnGa_{1.955}Cr_{0.005}Gd_{0.04}O₄.

La taille des nanoparticules est préférentiellement comprise entre 1 et 10³ nm.

### DESCRIPTION DE L'INVENTION

L'invention a pour objet des méthodes d'imagerie optique *in vivo* d'un organisme humain ou animal comprenant les étapes suivantes :
b) Excitation de la luminescence persistante de nanoparticules selon les revendications par irradiation *in vivo,* dans une longueur d'onde comprise entre 550 et 1000nm, de tout ou partie de l'organisme humain ou animal, lesdites nanoparticules ayant été préalablement administrées à l'organisme humain ou animal et lesdites nanoparticules émettant des photons à des longueurs d'onde comprises entre 550 et 1000nm pendant au moins 0.01 seconde, après une excitation lumineuse à des longueurs d'onde comprises entre 550 et 1000nm; et
c) Détection *in vivo* des nanoparticules dans tout ou partie de l'organisme humain ou animal par mesure de la luminescence persistante des nanoparticules par imagerie optique.

Dans des modes de réalisation particuliers de l'invention, l'utilisation de nanomatériaux ayant à la fois les propriétés de luminescence persistante après excitation dans le visible entre 550-1000nm et des propriétés paramagnétiques, permet l'utilisation de deux modalités d'imagerie, l'imagerie optique et l'IRM, pour la détection des nanoparticules *in vivo.*

L'invention concerne aussi des méthodes d'imagerie bimodale *in vivo* d'un organisme humain ou animal comprenant les étapes suivantes :
a) Excitation de la luminescence persistante de nanoparticules selon les revendications par irradiation *in vivo,* dans une longueur d'onde comprise entre 550 et 1000nm, de tout ou partie de l'organisme humain ou animal, lesdites nanoparticules ayant été préalablement administrées à l'organisme humain ou animal et lesdites nanoparticules émettant des photons à des longueurs d'onde comprises entre 550-1000nm pendant au moins 0.01 seconde, après une excitation lumineuse à des longueurs d'onde comprises entre 550 et 1000nm et lesdites nanoparticules ayant des propriétés paramagnétiques ;
b) Détection *in vivo* des nanoparticules dans tout ou partie de l'organisme humain ou animal par mesure de la luminescence persistante des nanoparticules par imagerie optique ; et
c) Détection *in vivo* des nanoparticules dans tout ou partie de l'organisme humain ou animal par imagerie par résonance magnétique.

De nombreux nanomatériaux ainsi que leurs propriétés optiques ont été décrits dans la littérature dont notamment des nanomatériaux ayant des propriétés de luminescence persistante.

Luminescence est le terme générique pour caractériser les substances qui vont restituer sous forme de photons d'origine non thermique une partie de l'énergie absorbée au cours d'une excitation non thermique. L'excitation de ces composés se fait par apport d'énergie qui peut prendre de nombreuses formes. On peut citer, sans être exhaustif, l'excitation par longueurs d'ondes de l'ultraviolet (UV), du visible ou de l'infrarouge (IR), par des rayonnements X, des réactions chimiques (chimiluminescence), des réactions enzymatiques (bioluminescence), des excitations électriques (électroluminescence) ou encore des excitations mécaniques (triboluminescence).

Les nanoparticules selon la présente invention sont sélectionnées pour leurs propriétés de luminescence persistante dans des intervalles de longueurs d'onde permettant à la fois l'excitation de la luminescence et la détection de la luminescence à travers les tissus d'un organisme humain ou animal.

Les nanoparticules selon la présente invention émettent des photons à des longueurs d'onde comprises entre 550 et 1000nm après une excitation lumineuse à des longueurs d'onde comprises entre 550 et 1000nm. De préférence, les nanoparticules émettent des photons après excitation à des longueurs d'onde comprises entre 550 et 800nm et plus préférentiellement entre 550 et 650 nm.

Les photons émis ont de préférence une longueur d'onde dans le proche infrarouge et plus particulièrement autour de 700nm.

Les nanoparticules selon l'invention émettent des photons pendant au moins 0,01 seconde. De préférence, les nanoparticules émettent des photons pendant au moins 1 seconde, de manière avantageuse pendant au moins 1 minute, 30 minutes, 1 heure, voire au moins 10 heures. Les temps de persistance sont évalués en suivant l'intensité de luminescence en fonction du temps après excitation. Des comparaisons claires de mesures des temps de persistance doivent être réalisées dans des conditions identiques en utilisant les mêmes systèmes de détection. L'expression "nanomatériau à luminescence persistante" a été appliquée à des nanomatériaux présentant une luminescence d'au moins 0,01 seconde jusqu'à plusieurs heures voire plusieurs jours.

Les nanoparticules émettant des photons pendant moins de 0,01 seconde ne sont pas comprises dans la présente invention car il s'agit alors de fluorescence distincte de la luminescence persistante.

Dans la présente demande, on entend désigner par "nanoparticule " une particule dont la taille, définie telle que la plus grande dimension selon un axe, est de manière générale comprise entre 1 et 1 000 nm et plus particulièrement de 10 à 1000 nm. De préférence, la nanoparticule selon l'invention est comprise entre 20 nm et 1000 nm, de manière encore préférée, entre 20 nm et 200 nm.

De préférence, la nanoparticule est constituée d'un nanomatériau d'une matrice choisie parmi :
- les gallates, les aluminates, les indates ;
- les oxysulfures, les oxysélenures et les oxytellures de zinc/magnésium et de gallium/aluminium/indium ;
- les oxydes de gallium, les oxydes d'indium, les oxydes de magnésium et leurs oxydes mixtes correspondants ;
- les composés mixtes tels que les gallogermanates, les aluminogallates ;

De préférence, les gallates sont choisis parmi ZnₓMg₍₁₋ₓ₎Ga₂O₄ avec x compris entre 0 et 1, LiGa₅O₈ et Ln₃Ga₅O₁₂ (Ln = Y, Gd, La ou Lu).

De préférence, les aluminates sont choisis parmi ZnₓMg₍₁₋ₓ₎Al₂O₄ avec x compris entre 0 et 1.

De préférence, les indates sont choisis parmi ZnₓMg₍₁₋ₓ₎In₂O₄ avec x compris entre 0 et 1.

De préférence, les oxydes sont choisis parmi Ga₂O₃, In₂O₃ et MgO.

De préférence, les gallogermanates sont choisis parmi M₃Ga₂Ge₄O₁₄ (M = Sr, Ca), ZnₓGa_{y}Ge_{z}O_{(x+(3y/2)+2z)} avec x, y et z intégrés de 1 à 5 et La₃Ga₅GeO₁₄.

De préférence, les aluminogallates sont choisis parmi ZnₓMg₍₁₋ₓ₎(Ga_{1-y}Al_{y})₂O₄ avec x compris entre 0 et 1, et y strictement compris entre 0 et 1 (ces deux valeurs étant exclues).

D'autres composés hybrides sont par exemple choisis parmi ZnₓMg(ᵢ_ₓ)(Gaᵢ-ylny)₂0₄ avec x compris entre 0 et 1, et y strictement compris entre 0 et 1 (ces deux valeurs étant exclues) ; La3GasSi014 et La₃Ga₅.₅Nbₒ.₅0₁₄.

Ces matrices sont dopées avec un métal de transition ou un lanthanide, de préférence choisi parmi le chrome, l'europium, le cérium, le nickel, le fer, le cuivre, et le cobalt.

Les nanomatériaux peuvent également comprendre des co-dopants pour améliorer les propriétés de luminescence de ces structures comme par exemple Li⁺ ; Na⁺ et Agi.

Les nanomatériaux peuvent comprendre tout autre co-dopant approprié.

Afin de conférer des propriétés magnétiques aux nanoparticules, les matrices sont dopées avec au moins un élément paramagnétique choisi parmi Cr³⁺, Mn ²+, Gd³⁺, Fe³⁺ et Ni³⁺.

Un nanomatériau préféré est le ZnGa₂₍₁₋ₓ₎Cr₂ₓO₄ avec x compris entre 0.001 et 0.0075 et plus préférentiellement le ZnGa_{1.995}Cr_{0.005}O₄.

Un autre nanomatériau préféré est le Zn₃Ga₂Ge₂O₁₀:0.5% Cr³⁺.

Parmi les nanomatériaux permettant l'imagerie bimodale optique et IRM, un nanomatériaux préféré est le ZnGa_{2(1-x-y)}Cr₂ₓGd₂yO₄ avec x compris entre 0.001 et 0.0075 et y compris entre 0.01 et 0.08 et plus préférentiellement le ZnGa_{1.955}Cr_{0.005}Gd_{0.04}O₄.

L'homme du métier est en mesure de déterminer quels matériaux à luminescence persistante peuvent être utilisés dans le cadre de la présente invention.

L'invention se rapporte aussi à des nanoparticules comprenant les nanomatériaux et les dopants décrits ci-dessus.

Les nanoparticules selon l'invention peuvent être utilisées en thérapie.

Les nanoparticules selon l'invention peuvent être utilisées en tant que sondes optiques ou marqueurs pour l'imagerie optique *in vivo* sur un organisme humain ou animal. L'organisme animal est de préférence un mammifère et plus préférentiellement un petit mammifère tel que la souris, le rat, le lapin ou le cobaye.

Les nanoparticules et méthodes d'imagerie selon l'invention sont particulièrement adaptées à l'imagerie du réseau vasculaire d'un organisme humain ou animal.

L'administration des nanoparticules à l'organisme humain ou animal peut être effectuée selon toute technique appropriée. De préférence, l'administration est effectuée par voie intraveineuse, intra-artérielle, intramusculaire, intrapéritonéale, ou encore rétro-orbitale.

Dans le cadre de la présente invention et contrairement à l'art antérieur, il n'est pas nécessaire d'exciter la luminescence persistante des nanoparticules avant leur injection dans l'organisme humain ou animal.

Les nanoparticules et les méthodes d'imagerie de la présente invention reposent sur l'excitation des nanoparticules « *in situ* », après injection dans l'organisme humain et animal. Ainsi, il devient possible de répéter cette excitation à volonté au cours du temps et d'effectuer un véritable suivi en temps réel de la distribution et localisation des nanoparticules dans l'organisme humain ou animal jusqu'à l'altération de l'intégrité des nanoparticules.

L'excitation des nanoparticules est effectuée dans le visible à une longueur d'onde comprise entre 550 et 1000 nm et de préférence entre 550 et 800nm, plus préférentiellement entre 550 et 650 nm. De façon inattendue ces photons, relativement proches de la fenêtre de transparence des tissus, s'avèrent assez pénétrants pour permettre une excitation suffisante des nanoparticules *in vivo* à travers l'organisme humain ou animal. En outre, on n'observe pas de phénomène d'autofluorescence de telle sorte que le rapport signal sur bruit est très favorable et que le contraste obtenu en imagerie est de bonne qualité.

Cette excitation s'effectue donc par irradiation *in vivo* de tout ou partie de l'organisme humain ou animal. L'organisme humain ou animal est par exemple soumis à une diode électroluminescente ou à un halogène pendant quelques minutes.

Grâce à leur propriété de luminescence persistante, les nanoparticules sont détectées *in vivo* à travers les tissus de l'organisme humain ou animal par imagerie optique à l'aide d'un détecteur optique approprié (caméra ICCD par exemple).

Lorsque les nanoparticules ont des propriétés magnétiques ou paramagnétiques, elles peuvent également être utilisées en imagerie par résonance magnétique (IRM). Les nanoparticules jouent alors le rôle d'agent de contraste. Il devient alors possible de détecter les nanoparticules *in vivo* dans l'organisme humain ou animal par imagerie bimodale combinant l'imagerie optique et l'IRM.

Selon des modes de réalisation préférés de l'invention, l'étape d'excitation des nanoparticules puis les étapes de détection des nanoparticules par mesure de la luminescence persistante en imagerie optique et éventuellement aussi par imagerie IRM peuvent être répétées *in vivo* sur tout ou partie de l'organisme humain ou animal. Après extinction totale ou partielle de la luminescence persistante des nanoparticules, il devient donc possible de re-exciter cette dernière en s'affranchissant alors complètement des contraintes liées à la durée de cette luminescence et de la perte d'intensité de la luminescence au cours du temps.

Les nanoparticules et les méthodes d'imagerie selon l'invention permettent donc un suivi en temps réel des nanoparticules dans l'organisme humain ou animal et cela pendant plusieurs heures ou même plusieurs jours.

Ceci ouvre un vaste champ d'applications nouvelles puisqu'il est dès lors possible, après injection, de suivre *in vivo* et en temps réel la distribution et la localisation des nanoparticules dans l'organisme humain ou animal.

De façon générale, les nanoparticules selon l'invention peuvent être administrées sans traitement additionnel du nanomatériau à l'organisme humain ou animal.

Selon des modes de réalisation particuliers, les nanoparticules peuvent être par exemple enrobées, fonctionnalisées et/ou encapsulées. Ces opérations sont effectuées selon des techniques habituelles connues de l'homme du métier.

Il peut être important de pouvoir modifier l'état de surface des nanoparticules pour favoriser leur circulation au sein de l'organisme humain ou animal. En particulier, il peut être avantageux de masquer les charges ioniques à la surface des particules par un enrobage afin de favoriser la circulation des nanoparticules après injection systémique.

Selon un mode de réalisation particulier, la nanoparticule selon l'invention est fonctionnalisée par enrobage avec une molécule chimique, un polymère, par greffage d'une substance d'intérêt biologique ou chimique, et/ou par greffage d'un ligand permettant une liaison *in vivo* avec un récepteur ou une substance d'intérêt biologique ou chimique dans l'organisme humain ou animal.

En greffant un ligand de récepteurs biologiques ou de substances d'intérêt biologique sur la surface des nanoparticules, il est possible de cibler les nanoparticules vers un organe, un tissu ou des cellules particulières dans l'organisme humain ou animal. Ceci peut avoir un intérêt aussi bien pour le diagnostic que dans une optique thérapeutique.

La substance d'intérêt peut être par exemple une molécule chimique connue pour son affinité vis-à-vis de certains récepteurs cellulaires. Il peut encore s'agir d'un anticorps, d'une protéine, ou d'un acide nucléique. Les termes protéine, polypeptide ou peptide désignent indifféremment une séquence d'acides aminés, comme les peptides RGD ou PHSCN connus pour leur forte affinité vis-à-vis de certaines intégrines surexprimées dans plusieurs formes de cancer, ou leurs dérivés, par exemple un composé contenant une séquence d'acides aminés. De même, les termes acide nucléique, séquence nucléique ou d'acides nucléiques, polynucléotide, oligonucléotide, séquence de polynucléotides, séquence nucléotidique, sont employés indifféremment pour désigner un enchaînement précis de nucléotides, modifiés ou non, permettant de définir un fragment ou une région d'un acide nucléique, comportant ou non des nucléotides non naturels, et pouvant correspondre aussi bien à un ARN interférant (« siRNA »), un ADN double brin, un ADN simple brin que des produits de transcription desdits ADN. Ces acides nucléiques sont isolés de leur environnement naturel, et sont naturels ou artificiels.

La substance d'intérêt peut être en particulier un principe actif ou une molécule thérapeutique.

Pour contrôler la biodistribution des nanoparticules à luminescence persistante et les orienter vers une substance d'intérêt biologique ou chimique dans un organisme ou un tissu, il est nécessaire de fonctionnaliser leur surface par enrobage et/ou greffage d'un ligand capable de se lier à la substance, le cas échéant présente au niveau tissulaire de l'organe d'intérêt.

Les méthodes d'enrobage sont bien connues de l'homme de l'art. Par exemple, l'enrobage peut être effectué par liaison avec des molécules portant des groupes phosphates, carboxylates ou thiols, ou encore par hétéroprécipitation de silice, d'aminosilane, ou de préférence de triéthoxyaminopropylsilane. L'enrobage effectué avec du triéthoxyaminopropylsilane offre l'avantage de ne former qu'une seule couche, sans polymérisation vers l'extérieur, limitant ainsi l'augmentation du diamètre des nanoparticules.

De même, les méthodes de greffage (ou couplage) du ligand sont bien connues de l'homme de l'art. Il s'agit en général d'un couplage par liaison covalente, par affinité, par adsorption passive ou forcée. Dans le cas d'un couplage par liaison covalente, les nanoparticules sont porteuses de groupements chimiques capables de réagir avec un autre groupement chimique porté par le ligand pour former une liaison covalente. Comme exemple de groupements chimiques pouvant être présents à la surface des nanoparticules on peut citer, mais sans s'y limiter, les acides carboxyliques, les amines, aldéhydes et époxy. On peut également utiliser l'interaction par affinité, qui est généralement mise en oeuvre par deux partenaires d'un couple de liaison à haute affinité tels que notamment, mais sans s'y limiter, les couples (poly) carbohydrate/lectine, biotine ou composés biotinilés/avidine ou streptavidine, récepteur /ligand spécifique, antigène ou haptène/anticorps, etc...

Le greffage des nanoparticules peut être réalisé directement, ou en utilisant des bras espaceurs encore désignés sous les termes "linker" ou "spacer".

Le couplage par adsorption passive ou forcée est connu de l'homme de l'art. On pourra utiliser par exemple la biotine-BSA (« Bovin Sérum Albumin »).

L'enrobage peut être réalisé par encapsulation de la nanoparticule dans un vecteur synthétique non viral comme les liposomes, les lipoplexes ou toute autre structure lipidique souple.

De préférence, l'enrobage est réalisé par précipitation de surface de triéthoxy aminopropylsilane, par polymérisation de monomères ou adsorption de polymères à la surface des nanoparticules.

Selon un autre mode préféré, on greffe du polyéthylène glycol (PEG) à des fins de furtivité (pour obtenir un temps de circulation dans l'organisme plus important). On procède généralement de la manière suivante : le PEG est d'abord couplé au ligand, puis le produit du couplage est greffé sur la nanoparticule.

Un enrobage ou le greffage en surface des nanoparticules peut également présenter l'intérêt de masquer les charges ioniques à la surface des particules.

De manière encore plus préférée, la nanoparticule selon l'invention est fonctionnalisée par précipitation à la surface de triéthoxyaminopropylsilane puis greffage de méthoxy-PEGₓ-COOH (x compris entre 0,2 et 20 kDa) permettant une liaison avec la substance d'intérêt biologique ou chimique.

En outre, le toluène peut être remplacé par du diméthylformamide, permettant ainsi une meilleure dispersion des particules. Les particules peuvent être fonctionnalisées par des groupements carboxylates (par réaction d'anhydride diglycolique sur les particules aminées) mais aussi par des groupements thiol après réaction directe avec du 3-mercaptopropyl-triéthoxysilane. Le greffage de polyéthylène glycol (PEG) peut être directement réalisé par couplage peptidique. Il également possible de greffer différentes molécules chimiques à la surface des nanoparticules (biotine, peptide).

Selon un mode de réalisation préféré, la nanoparticule selon l'invention est fonctionnalisëe par des groupements carboxylates, thiol ou amine libre.

Les nanoparticules selon l'invention peuvent également être utilisées comme sondes optiques ou marqueurs pour marquer des macromolécules ou des cellules avant une injection dans un organisme humain ou animal. La distribution en temps réel in vivo des molécules marquées avec les nanoparticules est ensuite suivie par imagerie optique et éventuellement aussi par imagerie IRM.

L'imagerie optique *in vivo* en temps réel de macrophages marqués avec des nanoparticules selon l'invention est décrite dans les exemples.

Les nanoparticules selon l'invention peuvent donc être utilisées pour marquer des cellules et suivre leur localisation et distribution *in vivo* au cours du temps après leur injection dans un organisme humain ou animal. Des applications diverses peuvent donc être envisagées dans le domaine de la thérapie cellulaire par exemple et en particulier dans des essais cliniques afin de suivre le devenir des cellules injectées.

Dans d'autres modes de réalisation les nanoparticules selon l'invention peuvent être encapsulées dans une coquille de silice mésoporeuse selon des techniques décrites dans la littérature. Des nanoparticules possédant une telle enveloppe de silice mésoporeuse et leurs procédés de préparation sont par exemples décrites par Kang et al. (2011), Kim et al. (2008) et Xu et al. (2011).

La silice mésoporeuse entourant les nanoparticules ne compromet pas leurs propriétés de luminescence persistante ni leurs éventuelles propriétés paramagnétiques. Cette couche poreuse permet le chargement et le relargage de substances d'intérêt biologique ou chimique.

La substance d'intérêt peut être par exemple une molécule chimique telle qu'un principe actif, une molécule thérapeutique ou bien une substance cytotoxique. Il peut encore s'agir d'une hormone, d'une protéine, d'un acide nucléique. Les termes protéine, polypeptide ou peptide désignent indifféremment une séquence d'acides aminés ou, pour leurs dérivés, un composé contenant une séquence d'acides aminés. De même, les termes acide nucléique, séquence nucléique ou d'acides nucléiques, polynucléotide, oligonucléotide, séquence de polynucléotides, séquence nucléotidique, sont employés indifféremment pour désigner un enchaînement précis de nucléotides, modifiés ou non, permettant de définir un fragment ou une région d'un acide nucléique, comportant ou non des nucléotides non naturels, et pouvant correspondre aussi bien à un ARN interférant (« siRNA »), un ADN double brins, un ADN simple brin, qu'à des produits de transcription des dits ADN ou ARN. Ces acides nucléiques sont isolés de leur environnement naturel, et sont naturels ou artificiels.

Les nanoparticules trouvent ainsi de nombreuses applications en diagnostic, en thérapie, et en théranostique.

L'imagerie vasculaire est, par exemple, intéressante pour la mise en évidence de processus angiogéniques dans l'inflammation chronique, la croissance tumorale ou la localisation des métastases. Les études de biodistribution sont, par ailleurs, indispensables pour déterminer les rapports d'accumulation d'un agent vectorisé ou non par des formes particulaires au niveau des tissus cibles. De manière encore préférée, l'agent de diagnostic est destiné à l'imagerie de zones tumorales, inflammatoires, de la rétine, lesdites zones étant susceptibles d'être hypervascularisées, ou encore des zones de rupture de la barrière hématoencéphalique. L'agent de diagnostic est destiné à l'imagerie de zones hypovascularisées telles que dans le cas d'une ischémie cérébrale ou cardiaque, ou dans le cas d'un trauma crânien. Une autre utilisation est la détection *in vivo* des zones inflammatoires et tumorales qui sont des zones hypervascularisées. Une application particulière est donc par exemple la détection précoce des cancers.

L'agent de diagnostic permet de mimer la biodistribution de liposomes ou de nanovecteurs *in vivo* dans une stratégie de thérapie génique.

De façon générale, l'invention se destine en particulier au domaine de la recherche biologique, médicale et clinique en tant que sonde optique, ou sonde multimodale ayant des propriétés simultanées de marqueur optique et agent de contraste pour l'IRM. Les exemples rapportent une application de ces sondes optiques pour le suivi cellulaire en temps réel *in vivo* dans un organisme humain ou animal. Les nanoparticules peuvent ainsi être utilisées comme marqueurs cellulaires pour un suivi en temps réel de la biodistribution de certains types cellulaires. Ces techniques permettent aussi la visualisation optique de la prise de greffons dans le cadre de thérapies cellulaires qui soulèvent un intérêt grandissant au sein de la communauté scientifique et médicale.

De par ses propriétés uniques de luminescence persistante, ces nanoparticules ouvrent également le champ à des applications en peropératoire comme aide aux chirurgiens pour la résection de tumeurs. Dans ce cas précis, les nanoparticules pourront être utilisées comme marqueurs de cellules tumorales, dont le métabolisme et la capacité d'endocytose sont augmentés, afin de guider et faciliter la résection des métastases et foyers tumoraux dans le cas de certains cancers, notamment pour les tumeurs du sein et de la vessie.

La présence d'une couche mésoporeuse autour de la nanoparticule à luminescence persistante ouvre le champ à d'autres applications, notamment la théranostique, d'autres fonctions, ou encore d'autres modalités d'imagerie. Les nanoparticules et méthodes selon l'invention permettent l'observation/le diagnostic et le traitement conjoints (théranostique) d'une pathologie ou bien de réaliser le suivi par imagerie optique de l'efficacité d'un traitement.

La présente invention a pour objet les nanoparticules telle que définies précédemment pour leur utilisation en tant qu'agent de diagnostic destiné à l'imagerie optique *in vivo.*

Cependent, les sujets non décrits dans les revendications ne font pas parti de la présente invention.

### FIGURES

**Figure 1**: Diffractogramme des composés ZnGa_{1.995}Cr_{0.005}O₄ (gauche) et ZnGa_{1.955}Cr_{0.005}Gd_{0.04}O₄ (droite). Les traits verticaux indiquent les pics de diffraction références du ZnGa₂O₄ (code référence : 00-038-1240).
**Figure 2** : Spectre d'émission de la lampe LED utilisée pour l'excitation visible (orange-rouge) des nanoparticules (A). Spectre d'excitation de la photoluminescence à 705 nm du composé ZnGa_{1.995}Cr_{0.005}O₄ (B). Spectre d'émission de la luminescence persistante après excitation dans l'UV et le visible (C). Déclins comparés de luminescence persistante après excitation en UV ou par le système LED (D).
**Figure 3**: Déclins comparés de luminescence persistante suivant la composition du matériau et le type d'excitation.
**Figure 4** : Cliché de microscopie électronique des nanoparticules de ZnGa_{1.995}Cr_{0.005}O₄ (le trait en gras représente 80 nm).
**Figure 5** **:** Schéma de fonctionnalisation des nanoparticules de ZnGa_{2(1-x-y)}Cr₂ₓGd₂yO_{4.}
**Figure 6** **:** Image du haut: A gauche, souris injectée avec des particules hydroxylées (de charge négative). A droite, souris injectée avec des particules PEGylées (neutres). Images du bas: A gauche, image optique d'une souris portant trois tumeurs CT26 en sous cutané et injectée (en intraveineux) avec les nanoparticules de ZnGa_{1.995}Cr_{0.005}O₄. A droite, signal de luminescence persistante obtenu après illumination de l'animal sous une source de lumière orange-rouge (voir Figure 2.A), 4 heures après injection de la sonde.
**Figure 7** **:** Images *in vivo* de la biodistribution de nanoparticules furtives à différents temps. La souris a été injectée avec 2 mg de nanoparticules PEGylées de 80 nm de diamètre de coeur. Les temps longs sont accessibles par ré-excitation de la luminescence persistante des particules pendant 2 minutes sous une source de lumière orange-rouge (voir Figure 2.A) à travers les tissus de l'animal.
**Figure 8** **:** Acquisition en luminescence persistante du signal provenant d'une suspension de macrophages RAW marqués par les nanoparticules à luminescence persistante.
**Figure 9** **:** Biodistribution *in vivo* comparée 30 minutes après injection systémique de nanoparticules à luminescence persistante aminées (gauche) et de cellules RAW (macrophages murins) marquées avec les mêmes nanoparticules à luminescence persistante (droite). Ces images sont enregistrées après excitation de la luminescence persistante dans le visible (entre 550 et 700 nm).
**Figure 10** **:** Biodistribution *ex vivo* 15 heures après injection systémique de nanoparticules aminées (rangée du haut) et de cellules RAW (macrophages murins) marquées avec les nanoparticules à luminescence persistante (rangée du bas). Ces images sont enregistrées après excitation des organes disséqués dans le visible pendant 2 minutes. On distingue une localisation majoritaire des cellules dans les poumons qui confirme la biodistribution *in vivo* de la Figure 9.
**Figure 11** **:** Image IRM obtenue en contraste T1 pour différentes concentrations (mg/mL) de nanoparticules de ZnGa_{1.995}Cr_{0.005}O₄ (gauche) et ZnGa_{1.955}Cr_{0.005}Gd_{0.04}O₄ (droite).
**Figure 12** **:** Image IRM obtenue en contraste T2 pour différentes concentrations (mg/mL) de nanoparticules de ZnGa_{1.995}Cr_{0.005}O₄ (gauche) et ZnGa_{1.955}Cr_{0.004}Gd_{0.04}O₄ (droite).
**Figure 13** **:** Relaxivités obtenues à partir des images IRM en contraste T1 et T2 avec nanoparticules de ZnGa_{2(1-x-y)}Cr₂ₓGd_{2y}O₄.

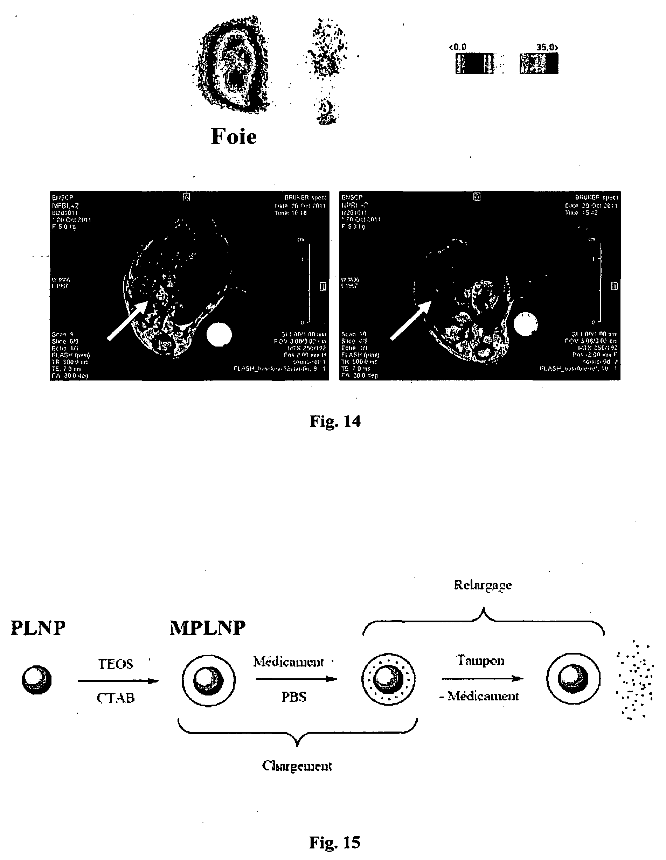

**Figure 14** **:** Image du haut: Suivi par imagerie optique de la biodistribution des nanoparticules non fonctionnalisées dopées au gadolinium, 2 mg de ZnGa_{1.955}Cr_{0.005}Gd_{0.04}O₄, 24h après injection. L'acquisition du signal est faite pendant 5 minutes après 2 minutes d'exposition de la souris à une source halogène émettant entre 550 et 700 nm. Images du bas: Coupe IRM (7T) du foie d'une souris référence sans particules (flèche rouge, image de gauche), et de la souris injectée avec le composé dopé au gadolinium (flèche rouge, image de droite). Les images IRM sont obtenues par pondération T2*. On observe un contraste négatif homogène au niveau de la masse du foie (zone foncée sur l'image de droite par rapport au foie témoin sans particules de gauche).
**Figure 15** **:** Principe d'utilisation de la structure mésoporeuse à luminescence persistante. PLNP : «persistent luminescence nanoparticles » ; MPLNP : « mesoporous persistent luminescence nanoparticles ».
**Figure 16** **:** Cliché de microscopie électronique de ZnGa_{1.995}Cr_{0.005}O₄ (partie foncée) après formation de la couche mésoporeuse (partie claire). Le trait en gras représente 100 nm.
**Figure 17****:** Paramètres de porosité des nanoparticules mésoporeuses à luminescence persistante (diamètre d'environ 100 nm de moyenne).
**Figure 18** **:** Cinétique de relargage de la doxorubicine après chargement de la DOX dans les nanoparticules mésoporeuses à luminescence persistante.
**Figure 19** **:** Test MTT après 24 h de contact entre des particules mésoporeuses sans doxorubicine (MPLNP), ou chargée avec la doxorubicine (MPLNP-Dox), et deux lignées de cellules cancéreuses. U87MG : lignée humaine de glioblastome ; CT26 : lignée murine de carcinome du côlon.
**Figure 20****:** Suivi par imagerie optique de la biodistribution des nanoparticules mésoporeuses non fonctionnalisées, 24h après injection de 2 mg de MPLNP. L'acquisition du signal est faite pendant 5 minutes après 2 minutes d'exposition de la souris à une source LED (voir Figure 2.A pour le spectre d'émission).

### EXEMPLES

### 1- Synthèse de ZnGa_{2(1-x-y)}Cr₂ₓGd₂yO₄ avec x ∈ [0.001; 0.0075] ; y ∈ [0.01 ; 0.08]

Les nanoparticules sont préparées par synthèse hydrothermale. Un mélange de nitrates de gallium, de chrome, de zinc, et de gadolinium dans les proportions voulues, adaptées à la composition souhaitée, est dissous dans l'eau sous agitation et à température ambiante. L'ajout d'ammoniac concentré à cette solution de cations, jusqu'à la valeur de pH = 7.5, permet la précipitation d'un précurseur de gallate de zinc. La suspension est ensuite agitée à température ambiante pendant 3 heures puis transvasée dans un réacteur en téflon pour subir un traitement sous pression à 120°C pendant 24 heures. Le composé obtenu après traitement est lavé plusieurs fois à l'eau et l'éthanol puis séché sous vide. Enfin, le composé séché est broyé en fine poudre puis calciné à 750°C pendant 5 heures.

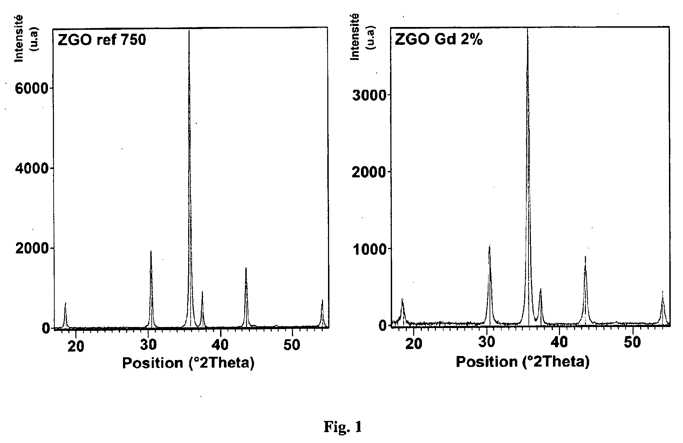

La structure du cristal est confirmée par diffraction des rayons X. Nous remarquons sur la Figure 1 que le composé sans gadolinium, ZnGa_{1.995}Cr_{0.005}O₄, et le composé avec gadolinium, ZnGa_{1.955}Cr_{0.005}Gd_{0.04}O₄, présentent la structure du gallate de zinc: ZnGa₂O₄. Nous remarquons également que l'ajout de ces deux cations (Cr³⁺ et Gd³⁺) n'induit aucune formation de phase parasite.

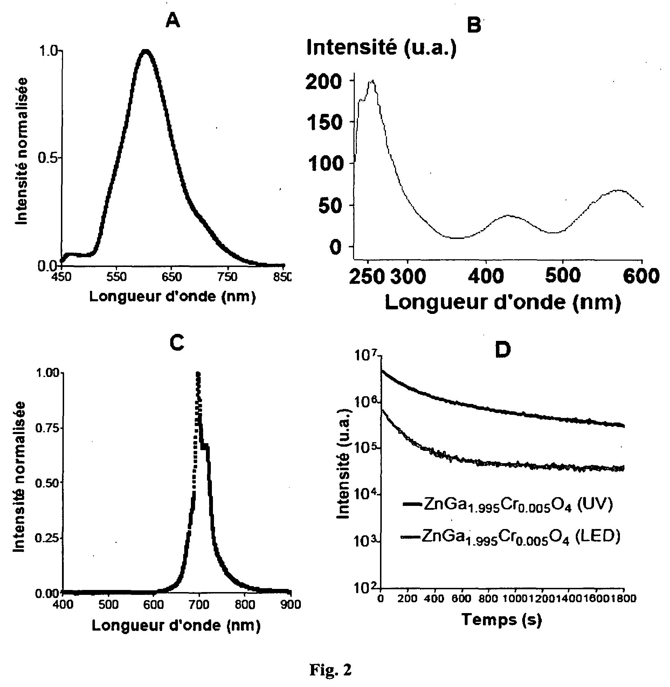

Les propriétés optiques des nanoparticules obtenues sont présentées en Figure 2. Nous remarquons qu'une excitation centrée sur 605 nm (Figure 2.A) permet d'activer le signal de luminescence persistante, plus faible, mais caractérisé par un spectre d'émission centré autour de 700 nm (Figure 2.C) et une cinétique de décroissance (Figure 2.D) similaires à ceux obtenus après excitation UV. Le spectre d'excitation de la photoluminescence à 705 nm du composé ZnGa_{1.995}Cr_{0.005}O₄ (Figure 2.B).

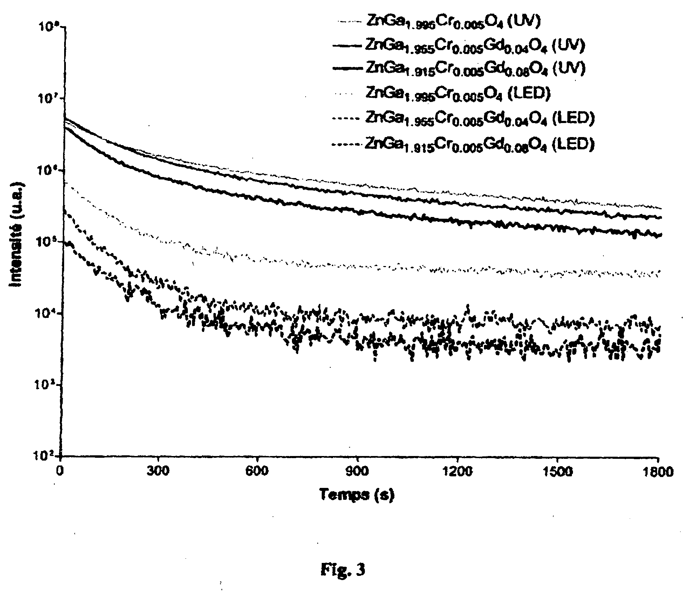

L'ajout de gadolinium induit une baisse de la luminescence persistante mais permet de conserver le même type de déclin, ainsi que des cinétiques comparables (Figure 3).

### 2 - Extraction d'une suspension monodisperse de nanoparticules de ZnGa_{2(1-xy)} Cr₂ₓGd_{2y}O₄

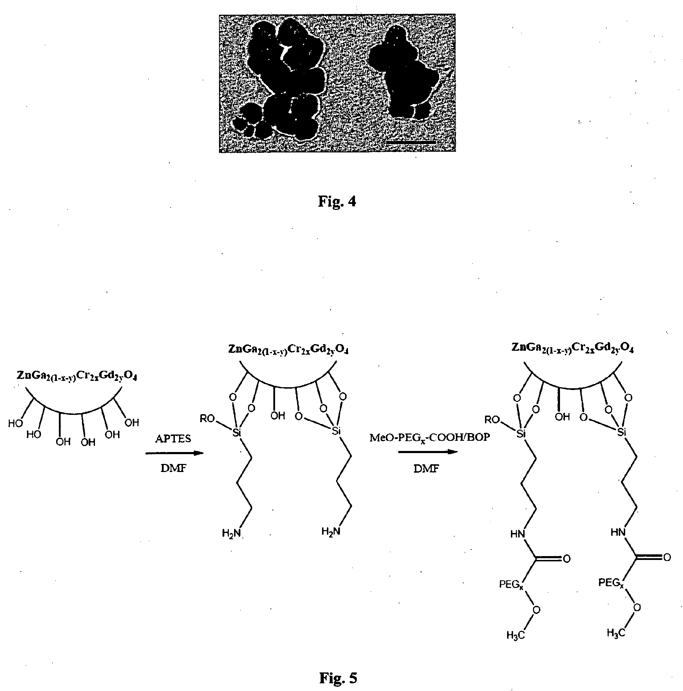

Les nanoparticules obtenues sont broyées pendant une dizaine de minutes puis reprises dans une solution de soude (5mM) pour permettre l'hydroxylation de surface. La suspension est passée au bain à ultrasons puis agitée pendant une nuit. Enfin, les nanoparticules sont extraites par centrifugations sélectives. Un ajustement précis des paramètres de centrifugation permet de sélectionner le diamètre d'intérêt. En exemple de nanoparticules de 80 nm de diamètre est donné en Figure 4.

### 3 - Fonctionnalisation de nanoparticules de ZnGa_{2(1-x-y)}Cr₂ₓGd_{2y}O₄

Après extraction d'une suspension monodisperse, il est important de pouvoir modifier l'état de surface des nanoparticules pour favoriser leur circulation au sein de l'organisme ou permettre un éventuel ciblage par ajout de ligands de récepteurs biologiques. Cette fonctionnalisation est possible par la réalisation successive de plusieurs modifications chimiques en surface des nanoparticules à luminescence persistante (Figure, 5) et permet de contrôler la biodistribution des sondes après injection.

Plusieurs étapes de caractérisation permettent d'assurer que la fonctionnalisation a réellement eu lieu. Une mesure de potentiel permet d'évaluer la charge de surface de ces nanoparticules à luminescence persistante. En particulier, l'ajout de polyéthylène glycol (PEG) permet de masquer les charges de surface et d'augmenter le temps de circulation des nanoparticules après injection systémique chez le petit animal.

### Détails et protocoles des étapes de fonctionnalisation

### Fonctionnalisation des nanoparticules par le 3-aminopropyltriethoxysilane (APTES) :

Les nanoparticules hydroxylées, en suspension dans le diméthylformamide (DMF) à la concentration de 2.5 mg/mL, sont dispersées dans un bain à ultrasons pendant 5 minutes. L'APTES est ensuite ajouté à la concentration volumique de 5%. La suspension est de nouveau laissée au bain à ultrasons pendant 5 minutes, puis placée sous forte agitation pendant 5h. Les nanoparticules sont finalement lavées plusieurs fois dans l'éthanol pour retirer l'excès d'APTES.

### Fonctionnalisation des nanoparticules par le polyéthylène glycol (PEG) :

Les nanoparticules aminées sont reprises dans le DMF à la concentration de 2.5 mg/mL puis dispersées dans un bain à ultrasons pendant 5 minutes. Une solution de PEG 5kDa (alpha-méthoxy gamma-N-hydroxysuccinimide, 25mg) est ensuite ajoutée à la suspension de nanoparticules. Le mélange est de nouveau dispersé au bain à ultrasons pendant 5 minutes, puis placé sous forte agitation à 90°C pendant une nuit. Les nanoparticules sont finalement lavées plusieurs fois au DMF pour retirer l'excès d'APTES.

### 4 - Injection des nanoparticules de ZnGa_{1.995}Cr_{0.005}O₄ fonctionnalisées ou non, et application des nanoparticules furtives pour le ciblage de tumeurs CT26

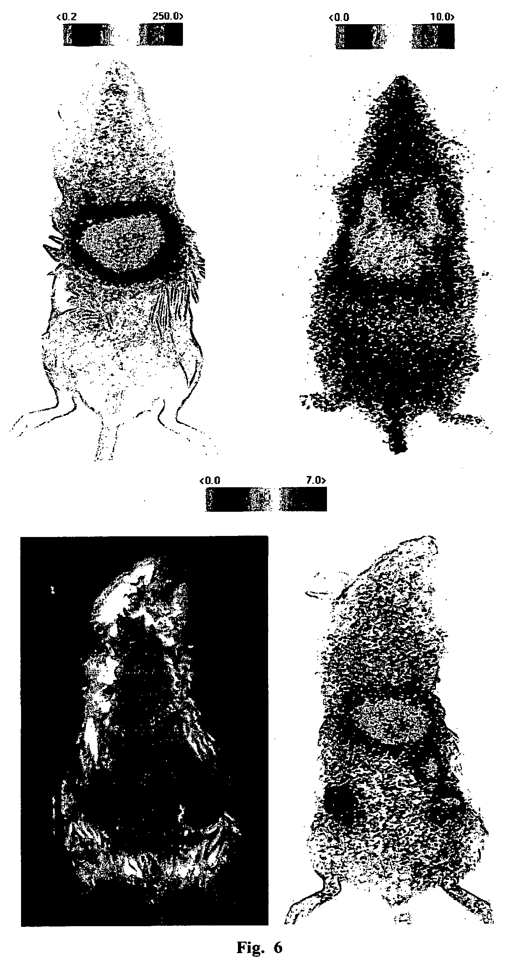

On remarque sur la Figure 6 (images du haut) une nette différence de biodistribution entre les nanoparticules négatives et celles dont la charge est masquée par le PEG. Cet effet est caractéristique de nombreux types de nanoparticules. Les nanoparticules négatives, du fait de leur charge de surface, subissent un processus rapide d'opsonisation puis de capture par les macrophages du foie (les cellules de Kupffer), qui apparaît sur l'image de gauche (Figure 6, image du haut). En revanche, ce processus de reconnaissance est retardé lorsque les charges de surface sont dissimulées par ajout du PEG. C'est ce que l'on constate sur l'image de droite, en haut de la Figure 6. Les particules sont réparties de manière plus homogène au sein de l'organisme, elles se distribuent dans la circulation générale.
L'injection intraveineuse de ces nanoparticules PEGylées, dites furtives, à une souris portant des tumeurs CT26 en sous cutané (Figure 6, en bas à gauche) permet de cibler la zone tumorale de manière passive, par accumulation progressive de la sonde au niveau des tissus malades. Le signal de luminescence persistante nous permet alors de nettement distinguer les tumeurs implantées à l'animal (Figure 6, en bas à droite).

### 5 - Excitation de la luminescence persistante des nanoparticules à travers le corps de l'animal

L'un des gros points faibles de la génération précédente de nanoparticules à luminescence persistante réside dans son incapacité à être excitée à travers les tissus de l'animal, limitant l'observation à une durée n'excédant pas une heure. Les acquisitions du signal de luminescence persistante présentées ci-dessous indiquent que ces composés (ZnGa_{2(1-x-y)}Cr₂ₓGd_{2y}O₄), sous forme de nanoparticules, peuvent être excités à travers les tissus de l'animal, de façon à rendre un signal de luminescence persistante. Cette innovation permet de se soustraire à toute contrainte de temps et d'observer les nanoparticules à tout moment.

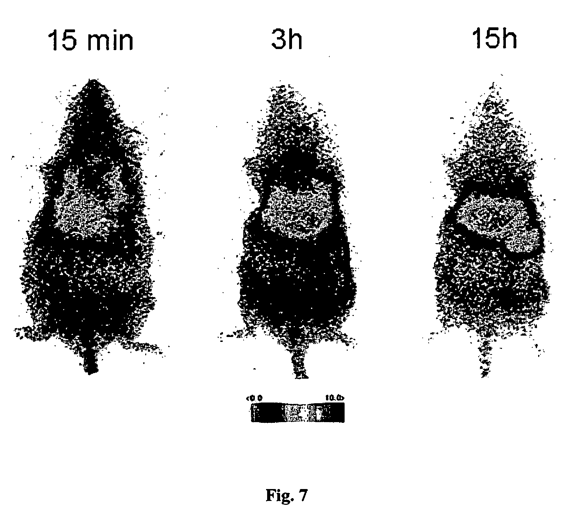

Pour cette manipulation, les souris sont anesthésiées avec un mélange de kétamine/xylazine puis injectées avec les nanoparticules de ZnGa_{1.995}Cr_{0.005}O₄ dans la veine de la queue. La souris est ensuite placée sous le système de LED (voir Figure 2.A pour le spectre d'émission), pendant 2 minutes pour exciter la luminescence persistante, puis disposées sous une caméra ICCD (système de compte de photons, Biospace Lab). Nous remarquons sur la Figure 7 que le signal de luminescence persistante permet de localiser les particules et de suivre leur biodistribution après plusieurs heures.

### 6 - Exemple d'application pour le suivi cellulaire en temps réel chez la souris

Nous rapportons également comme exemple original d'application de cette technologie, la possibilité de marquer des cellules au moyen de ces nanoparticules à luminescence persistante, et de suivre la biodistribution cellulaire après injection systémique chez le petit animal :

### Marquage des cellules RAW (macrophages murins) et injection :

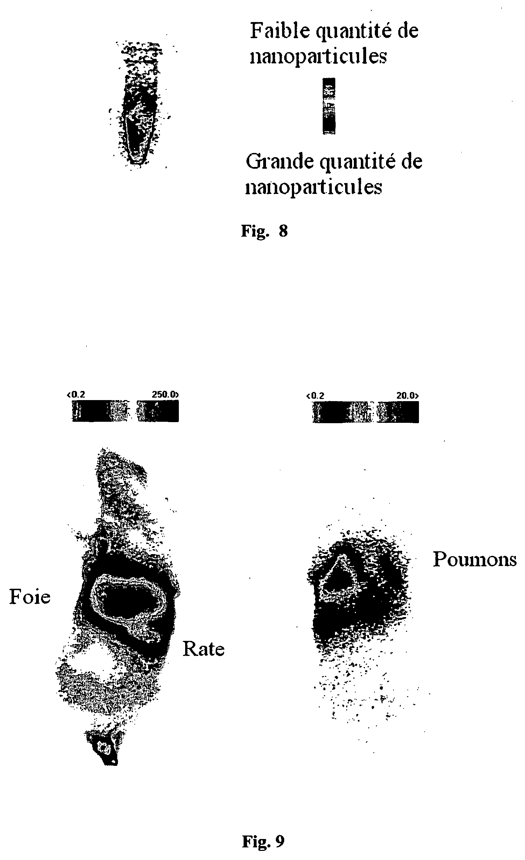

Le marquage des cellules se fait par incubation des macrophages RAW (10⁶ par puits, plaque 6 puits) avec 2 mL d'une suspension de nanoparticules dans du milieu de culture DMEM sans sérum (1 mg/mL) pendant 6h. Après incubation, les cellules sont lavées plusieurs fois avec le milieu de culture pour enlever l'excès de nanoparticules, reprises dans le même milieu, puis concentrées par centrifugation à 900 rpm pour injection à la souris (300 µL). On peut vérifier le marquage efficace des cellules par excitation de la luminescence persistante avec le système de LED (Figure 8).

La manipulation *in vivo* est réalisée en comparant la biodistribution de nanoparticules aminées, 2 mg en suspension dans le milieu de culture sans sérum, injectées dans la veine de la queue, à celle des macrophages marqués par les nanoparticules (10⁶ cellules) en suspension dans le même milieu. Les résultats sont présentés dans la Figure 9.

On constate une nette différence de biodistribution entre les particules seules et les macrophages marqués. Les particules seules se concentrent au niveau du foie, pour les même raisons que celles évoquée plus haut. Enfin, les cellules marquées se fixent dans les poumons.

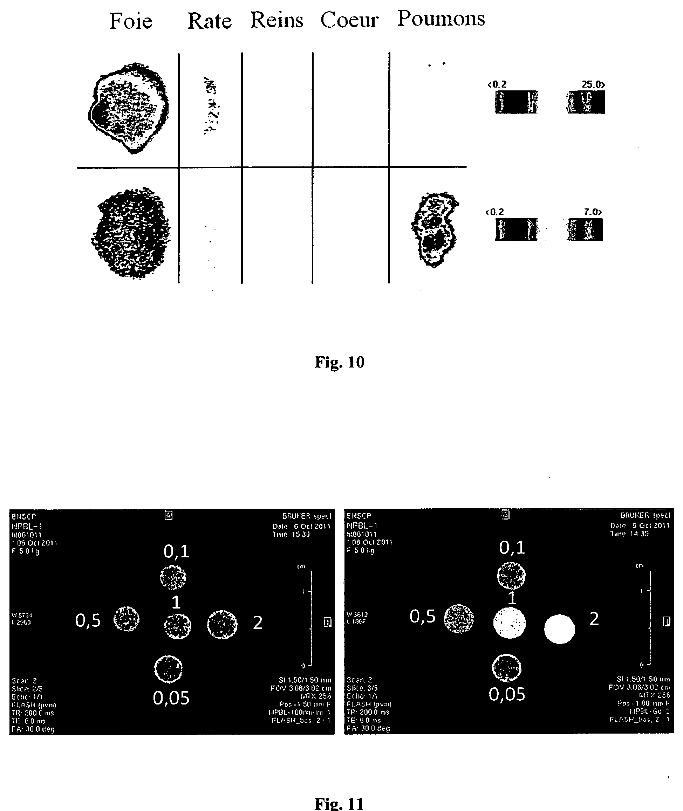

La ré-excitation à travers les tissus permet également de réaliser une quantification *ex vivo,* après prélèvement des organes (les conditions d'excitation sont les mêmes que précédemment, avec le système de LED). Les images de luminescence obtenues sont présentées en Figure 10.

### 7 - Exemple d'application de ZnGa_{2(1x-y)}Cr₂ₓGd_{2y}O₄ pour l'imagerie multimodale

Nous avons déjà précisé que l'ajout de gadolinium dans la structure du gallate de zinc ne change pas la nature des propriétés optiques. En particulier, le phénomène de luminescence persistante, certes moins intense, est conservé, ainsi que les propriétés d'excitabilité du matériau au-delà de 600 nm (système de LED, Figure 2.A).

Les nanoparticules utilisées pour la mesure des temps de relaxation (T1 et T2) *in vitro* sont préparées suivant le protocole décrit dans les deux premières parties. Les nanoparticules ont un diamètre d'environ 80 nm.

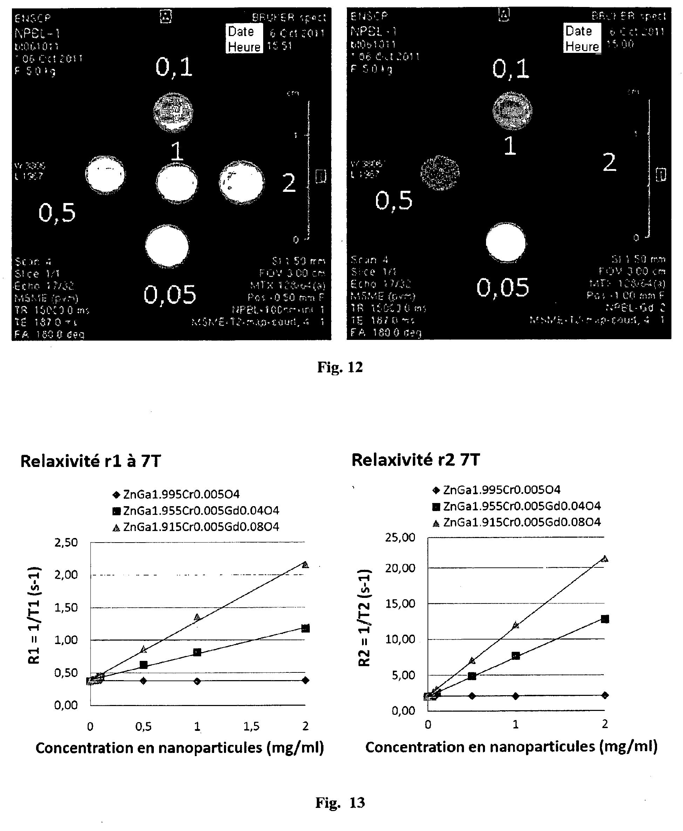

On constate sur les Figures 11 et 12 (images de gauche) que les nanoparticules non dopées au gadolinium ne présentent aucun effet de contraste dépendant de la concentration (même contraste au niveau des ronds) ainsi que des valeurs de relaxivité similaires à celles du témoin (sans nanoparticules). En revanche, les mesures de relaxivité R1 et R2, ainsi que les images obtenues en IRM 7T (Figure 11, 12, à droite ; puis Figure 13), indiquent un effet T1 et T2 des nanoparticules lié à l'ajout du gadolinium. Cet effet est bien dépendant de la concentration en nanoparticules : plus la concentration est importante, plus le contraste est marqué.

Des comparaisons avec les valeurs de relaxivité des agents commerciaux T1 et T2 (Dotarem et Endorem) nous permettent de dire, à la vue des relaxivités calculées pour les nanoparticules de ZnGa_{2(1-x-y)}Cr_{2y}Gd_{2y}O₄, que l'effet T2 est prépondérant (la calcul du rapport R2/R1>1 permet de le dire).

### Exemple d'application in vivo, chez le petit animal :

Dans un premier temps, l'imagerie optique permet de localiser les particules après injection intraveineuse. L'accumulation des nanoparticules à luminescence persistante au niveau du foie est suivie par imagerie optique, 24 heures après injection (Figure 14, 2 mg de ZnGa_{1.955}Cr_{0.005}Gd_{0.04}O₄ de 80 nm de diamètre, en suspension dans le glucose 5% pour l'injection). L'acquisition IRM en contraste T2* nous permet de confirmer la présence des nanoparticules réparties de manière homogène dans la masse du foie (en comparaison du foie témoin n'ayant reçu aucune particule). Ce type de contraste négatif diffus au niveau du foie est caractéristique d'une capture par les macrophages de l'organe, les cellules de Kupffer.

### 8-Synthèse des nanoparticules mésoporeuses à luminescence persistante (MPLNP)

Le principe d'utilisation des nanoparticules mésoporeuses à luminescence persistante pour des applications en théranostique est résumé en Figure 15.

### Exemple de synthèse avec ZnGa_{1.995}Cr_{0.005}O₄ :

La couche mésoporeuse est formée par condensation de tétraéthoxysilane (TEOS) autour des nanoparticules en présence d'un surfactant cationique : le bromure de cétyltriméthylammonium (CTAB).

Les nanoparticules sont suspendues dans une solution de CTAB (4mg/mL) dans la soude 5 mM à la concentration de 1 mg/mL. Le mélange est bien dispersé au bain à ultrasons puis placé à 45°C sous forte agitation. Ensuite, le TEOS est ajouté au goutte à goutte à la suspension de nanoparticules pour obtenir une concentration finale de 1% en volume (ex : 10 µL de TEOS pour 1 mL de suspension). Après 3 heures d'agitation à 45°C, la suspension est transférée dans un réacteur en téflon pour subir un vieillissement sous pression pendant 24h à 100°C. La suspension est finalement lavée plusieurs fois à l'eau et l'éthanol pour enlever l'excès de surfactant.

La structure poreuse est obtenue par extraction du surfactant (CTAB) de la couche de silice qui enrobe les nanoparticules à luminescence persistante. Cette extraction se fait dans une solution de NaCl dans le méthanol (1% en masse). Les nanoparticules sont suspendues dans cette solution saline de méthanol puis agitées pendant 3h. Après extraction, les nanoparticules sont lavées plusieurs fois à l'éthanol. Cette étape d'extraction est répétée 3 fois de manière à s'assurer que tout le surfactant a bien été enlevé.

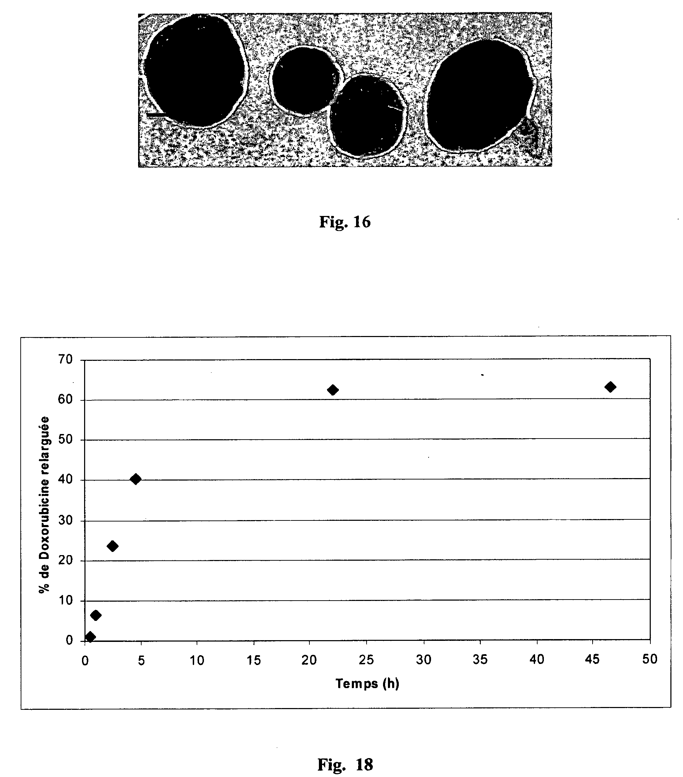

La figure 16 présente un cliché de microscopie électronique de la structure coeur-coquille obtenue après encapsulation des cristaux de gallates dans une couche de silice mésoporeuse. On distingue nettement le cristal au coeur de la structure, plus dense car moins transparent aux électrons, ainsi que la couche de silice amorphe, en clair sur le cliché du fait de sa transparence aux électrons.

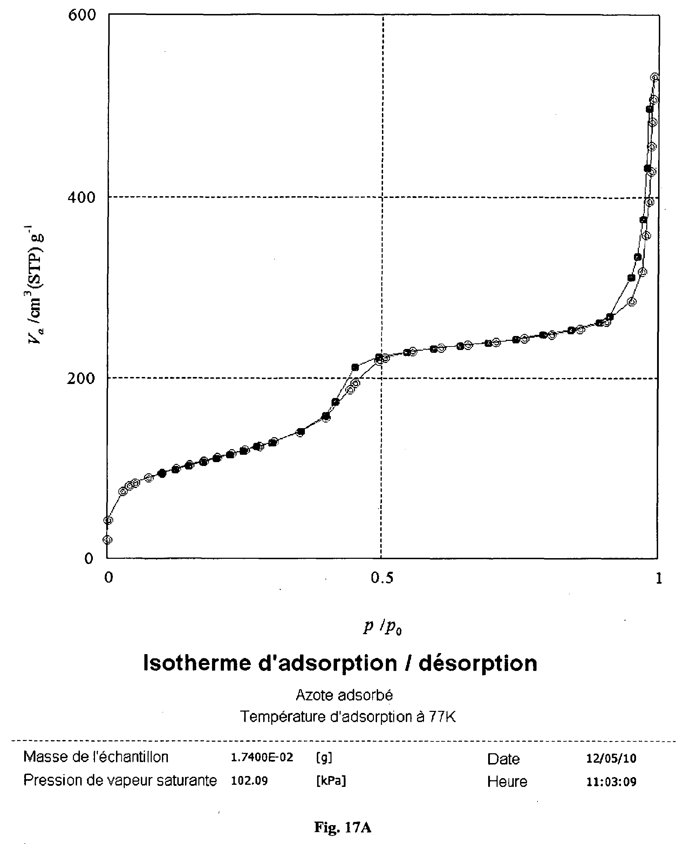

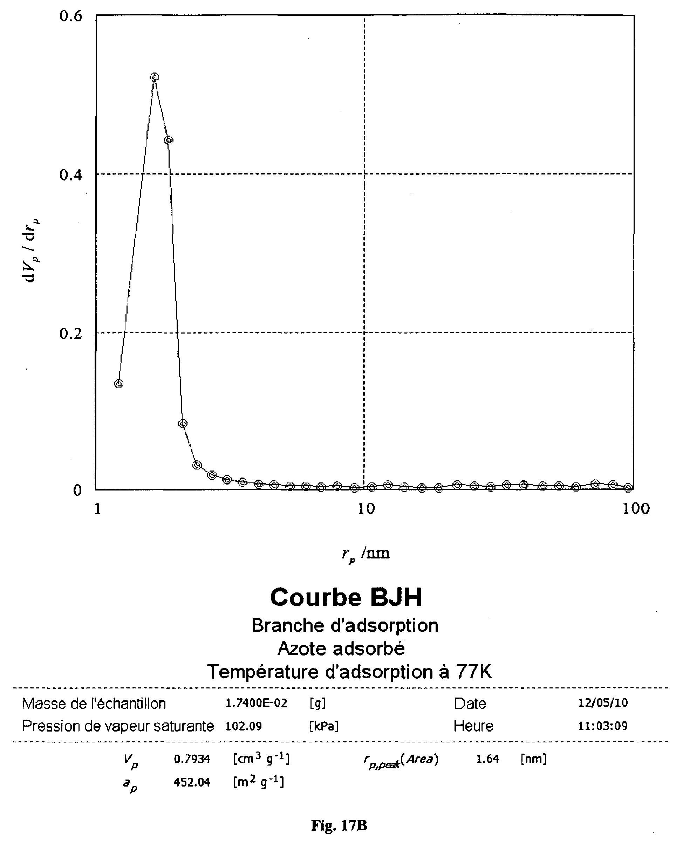

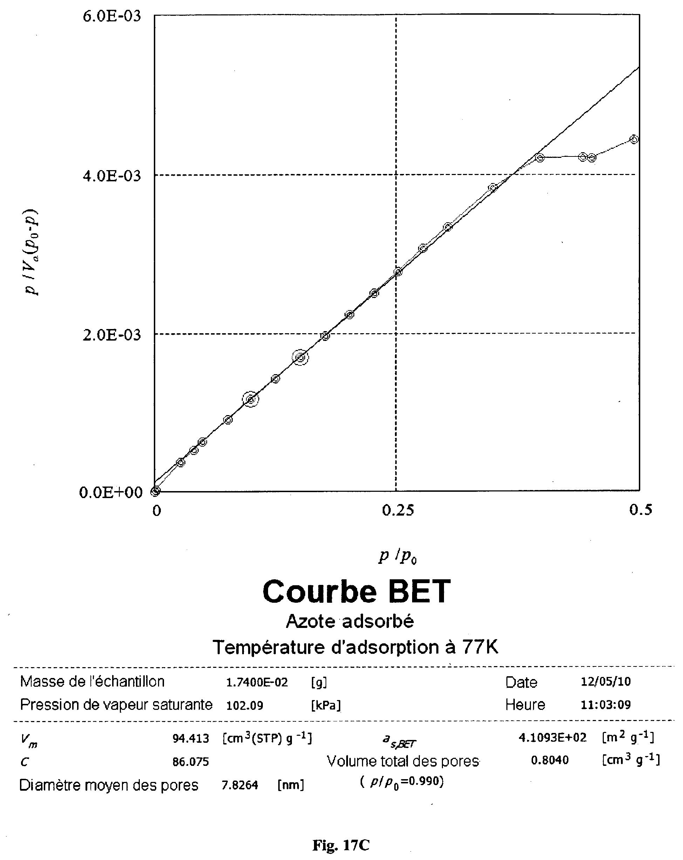

Les paramètres de porosité sont déterminés par adsorption d'azote à 77 K. Les résultats de la Figure 17 indiquent une surface spécifique accessible de 400 m²/g de composé. Le diamètre des pores est d'environ 2 nm.

### 9 - Exemple d'application des MPLNP pour la délivrance de molécules cytotoxiques

L'idée étant d'utiliser cette structure pour l'administration de principes actifs, nous avons réalisé une première preuve de concept avec la doxorubicine (Dox), utilisée en clinique pour le traitement de certains cancers. Cette molécule absorbe nettement la lumière autour de 480 nm. Pour cette raison, le chargement de la Dox dans la structure poreuse est suivi par mesure de l'absorbance à 480 nm. Nous avons évalué la quantité de Dox (par unité de masse de particules) à 150 µg par mg de nanoparticules mésoporeuses. La même technique de dosage peut être utilisée pour évaluer là cinétique de relargage du composé dans un tampon phosphate (Figure 18).

Dans le but d'évaluer la possibilité d'utiliser ces nanoparticules pour véhiculer et libérer une molécule cytotoxique, nous avons comparé la toxicité des nanoparticules chargées avec la Dox (MPLNP-Dox) à celle de nanoparticules non chargées (MPLNP) sur plusieurs lignées cellulaires (CT26 et U87MG).

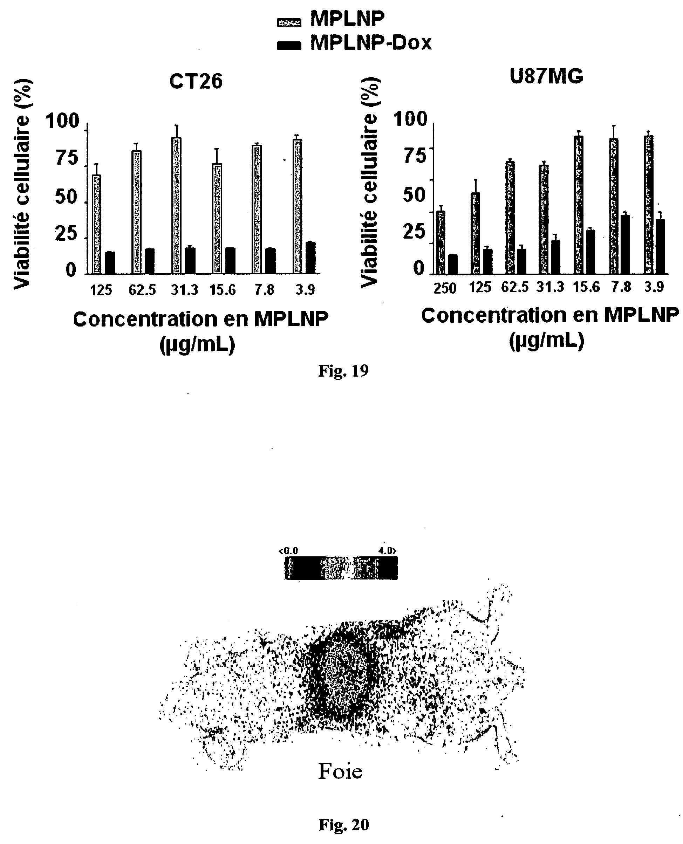

Protocole du test : Les cellules sont cultivées puis placées dans des plaques 96 puits à la concentration de 10000 cellules/puits. Le test de toxicité (MTT) est réalisé 24 heures après le dépôt de nanoparticules à différentes concentrations sur des cellules. Les résultats sont présentés sur la Figure 19. On remarque une nette toxicité des nanoparticules chargées avec la doxorubicine en comparaison des nanoparticules témoin (non chargées).

Enfin nous avons démontré *in vivo* que la formation de la couche de silice mésoporeuse sur ces nanoparticules à luminescence persistante n'empêchait pas l'excitation de la luminescence persistante à travers les tissus. L'image de la Figure 20 montre le signal de luminescence persistante obtenu après excitation visible (système de LED, voir Figure 2.A) d'une souris injectée avec les MPLNP.

### REFERENCES

### REFERENCES BREVETS

WO 2007/048856
FR 2 908 891

### REFERENCES NON BREVETS

- le Mase de Chermont, Quentin, Corinne Chanéac, Johanne Seguin, Fabienne Pellé, Serge Maîtrejean, Jean-Pierre Jolivet, Didier Gourier, Michel Bessodes, and Daniel Scherman. "Nanoprobes with near-infrared persistent luminescence for in vivo imaging." Proceedings of the National Academy of Sciences of the United States of America 104, no. 22 (May 29, 2007): 9266-71.
- Pan, Zhengwei, Yi-Ying Lu, and Feng Liu. "Sunlight-activated long-persistent luminescence in the near-infrared from Cr3+-doped zinc gallogermanates." Nature Materials 11, no. 1 (November 20, 2011): 58-63. http://www.nature.com/doifinder/10.1038/nmat3173.
- Bessière, Aurélie, Sylvaine Jacquart, Kaustubh Priolkar, Aurélie Lecointre, Bruno Viana, and Didier Gourier. "ZnGa2 O4:Cr 3+ : a new red long-lasting phosphor with high brightness." Optics Express 19, no. 11 (2011): 10131-10137.
- Liu, Yanlan, Kelong Ai, Qinghai Yuan, and Lehui Lu. "Fluorescence-enhanced gadolinium-doped zinc oxide quantum dots for magnetic resonance and fluorescence imaging." Biomaterials 32, no. 4 (February 2011): 1185-92. http://www.ncbi.nlm.nih.gov/pubmed/21055806.
- Kim, Jaeyun, Hoe Suk Kim, Nohyun Lee, Taeho Kim, Hyoungsu Kim, Taekyung Yu, In Chan Song, Woo Kyung Moon, and Taeghwan Hyeon. "Multifunctional Uniform Nanoparticles Composed of a Magnetite Nanocrystal Core and a Mesoporous Silica Shell for Magnetic Resonance and Fluorescence Imaging and for Drug Delivery." Angewandte Chemie 120, no. 44 (October 20, 2008): 8566-8569. http://doi.wiley.com/10.1002/ange.200802469.
- Kang, Xiaojiao, Ziyong Cheng, Chunxia Li, Dongmei Yang, Mengmeng Shang, Ping Ma, Guogang Li, Nian Liu, and Jun Lin. "Core-Shell Structured Up-Conversion Luminescent and as Carriers for Drug Delivery" (2011).
- Xu, Zhenhe, Yu Gao, Shanshan Huang, Ping' an Ma, Jun Lin, and Jiye Fang. "A luminescent and mesoporous core-shell structured Gd2O3: Eu(3+)@nSiO2@mSiO2 nanocomposite as a drug carrier." Dalton transactions (Cambridge, England: 2003) 40, no. 18 (May 14, 2011): 4846-54. http://www.ncbi.nlm.nih.gov/pubmed/21431226.
- J.S. Kim, J.S. Kim, H.L. Park, Solid State Communications 131 (2004) 735-738.

## Revendications

1. Méthode d'imagerie optique *in vivo* d'un organisme humain ou animal comprenant les étapes suivantes :
a) Excitation de la luminescence persistante de nanoparticules par irradiation *in vivo* dans une longueur d'onde comprise entre 550 et 1000nm de tout ou partie de l'organisme humain ou animal, lesdites nanoparticules ayant été préalablement administrées à l'organisme humain ou animal et lesdites nanoparticules émettant des photons à des longueurs d'onde comprises entre 550 et 1000nm pendant au moins 0.01 seconde, après une excitation lumineuse à des longueurs d'onde comprises entre 550 et 1000nm et les dites nanoparticules comprenant un nanomatériau constitué d'une matrice choisie parmi les gallates, les aluminates, les indates, et leurs composés mixtes gallogermanates, gallo-aluminates, gallo-indates, les oxydes de gallium, les oxydes d'indium, les oxydes de magnésium, les oxysulfures zinc et de gallium, les oxysélénures de zinc et de gallium, les oxytellures de zinc et de gallium, ladite matrice étant dopée avec un métal de transition ou un lanthanide choisi parmi le chrome, l'europium, le cérium, le nickel, le fer, le cuivre et le cobalt; et
b) Détection *in vivo* des nanoparticules dans tout ou partie de l'organisme humain ou animal par mesure de la luminescence persistante des nanoparticules par imagerie optique.

2. Méthode d'imagerie optique *in vivo* d'un organisme humain ou animal selon la revendication 1, dans laquelle les dites nanoparticules comprennent le nanomatériau ZnGa₂₍₁₋ₓ₎Cr₂ₓO₄ avec x compris entre 0.001 et 0.0075.

3. Méthode d'imagerie optique *in vivo* d'un organisme humain ou animal selon l'une des revendications 1-2, dans laquelle les dites nanoparticules comprennent le nanomatériau ZnGa_{1.995}Cr_{0.005}O₄.

4. Méthode d'imagerie optique *in vivo* d'un organisme humain ou animal selon l'une des revendications 1-3, dans laquelle l'administration des nanoparticules est préalablement effectuée par voie intraveineuse, intraartérielle, intramusculaire, intrapéritonéale ou rétro-orbitale.

5. Méthode d'imagerie optique *in vivo* d'un organisme humain ou animal selon l'une des revendications 1-4, dans laquelle les nanoparticules ont une taille comprise entre 1 et 10³ nm.

6. Méthode d'imagerie optique *in vivo* d'un organisme humain ou animal selon l'une des revendications 1-5, dans laquelle les nanoparticules sont greffées en surface, avantageusement avec un ligand, ou enrobées.

7. Méthode d'imagerie optique *in vivo* d'un organisme humain ou animal selon l'une des revendications 1-6, dans laquelle les nanoparticules sont encapsulées dans de la silice mésoporeuse permettant le chargement et le relargage de molécules d'intérêt.

8. Méthode d'imagerie bimodale *in vivo* d'un organisme humain ou animal comprenant les étapes suivantes :
a) Excitation de la luminescence persistante des nanoparticules par irradiation *in vivo* dans une longueur d'onde comprise entre 550 et 1000nm de tout ou partie de l'organisme humain ou animal, lesdites nanoparticules ayant été préalablement administrées à l'organisme humain ou animal et lesdites nanoparticules émettant des photons à des longueurs d'onde comprises entre 550-1000 nm pendant au moins 0.01 seconde, après une excitation lumineuse à des longueurs d'onde comprises entre 550 et 1000nm et lesdites nanoparticules ayant des propriétés paramagnétiques et les dites nanoparticules comprenant un nanomatériau constitué d'une matrice parmi les gallates, les aluminates, les indates , les oxydes de gallium, les oxydes d'indium, les oxydes de magnésium, les gallogermanates, les aluminogallates, les oxysulfures zinc et de gallium, les oxysélénures de zinc et de gallium, les oxytellures de zinc et de gallium, ladite matrice étant dopée avec un métal de transition ou un lanthanide choisi parmi le chrome, l'europium, le cérium, le nickel, le fer, le cuivre, et le cobalt et avec au moins un élément paramagnétique choisi parmi Cr³⁺; Mn²⁺ ; Gd³⁺; Fe³⁺ et Ni³⁺.;
b) Détection *in vivo* des nanoparticules dans tout ou partie de l'organisme humain ou animal par mesure de la luminescence persistante des nanoparticules par imagerie optique ; et
c) Détection *in vivo* des nanoparticules dans tout ou partie de l'organisme humain ou animal par imagerie par résonance magnétique.

9. Méthode d'imagerie bimodale *in vivo* d'un organisme humain ou animal selon la revendications 8, dans laquelle les dites nanoparticules comprennent le nanomatériau ZnGa_{2(1-x-y)}Cr₂ₓGd_{2y}O₄ avec x compris entre 0.001 et 0.0075 et y compris entre 0.01 et 0.08.

10. Méthode d'imagerie bimodale *in vivo* d'un organisme humain ou animal selon l'une des revendications 8-9, dans laquelle les dites nanoparticules comprennent le nanomatériau ZnGa_{1.955}Cr_{0.005}Gd_{0.04}O₄.

11. Méthode d'imagerie bimodale *in vivo* d'un organisme humain ou animal selon l'une des revendications 8-10, dans laquelle l'administration des nanoparticules est préalablement effectuée par voie intraveineuse, intraartérielle, intramusculaire, intrapéritonéale ou rétro-orbitale.

12. Méthode d'imagerie bimodale *in vivo* d'un organisme humain ou animal selon l'une des revendications 8-11, dans laquelle les nanoparticules ont une taille comprise entre 1 et 10³ nm.

13. Méthode d'imagerie bimodale *in vivo* d'un organisme humain ou animal selon l'une des revendications 8-12, dans laquelle les nanoparticules sont greffées en surface avantageusement avec un ligand ou enrobées.

14. Méthode d'imagerie bimodale *in vivo* d'un organisme humain ou animal selon l'une des revendications 8-13, dans laquelle les nanoparticules sont encapsulées dans de la silice mésoporeuse permettant le chargement et le relargage de molécules d'intérêt.

15. Nanoparticule comprenant le nanomatériau ZnGa_{2(1-x-y)}Cr₂ₓGd_{2y}O₄ avec x compris entre 0.001 et 0.0075 et y compris entre 0.01 et 0.08, en particulier le nanomatériau est ZnGa_{1.955}Cr_{0.005}Gd_{0.04}O₄.

16. Nanoparticule selon la revendication 15, **caractérisées en ce que** sa taille est comprise entre 1 et 10³ nm.

## Patentansprüche

1. Verfahren der optischen in-vivo-Bildgebung eines menschlichen oder tierischen Organismus, das die folgenden Schritte umfasst:
a) Erregung der persistenten Lumineszenz von Nanopartikeln durch in vivo-Bestrahlung in einer Wellenlänge zwischen 550 et 1000 nm inklusive des gesamten oder eines Teils des menschlichen oder tierischen Organismus, wobei die Nanopartikel dem Organismus zuvor verabreicht wurden und wobei die Nanopartikel nach Lichterregung auf Wellenlängen zwischen 550 und 1000 nm inklusive Photonen mit Wellenlängen zwischen 550 und 1000 nm inklusive während mindestens 0,01 Sekunde senden und wobei die Nanopartikel ein Nanomaterial umfassen, das aus einer Matrix besteht, die aus den Gallaten, den Aluminaten, den Indaten und ihren gemischten Gallo-Germanat-, Gallo-Aluminat-, Gallo-Indat-Zusammensetzungen, den Galliumoxiden, den Indiumoxiden, den Magnesiumoxiden, den Zink- und Galliumoxisulfiden, den Zink- und Galliumseleniden, den Zink- und Galliumoxitelluren ausgewählt ist, wobei die Matrix mit einem Übergangsmetall oder einem Lanthanid dotiert ist, das aus dem Chrom, dem Europium, dem Cerium, dem Nickel, dem Eisen, dem Kupfer und dem Kobalt ausgewählt ist; und
b) in vivo-Detektion der Nanopartikel im gesamten oder in einem Teil des menschlichen oder tierischen Organismus durch Messen der persistenten Lumineszenz der Nanopartikel durch optische Bildgebung.

2. Verfahren der optischen in-vivo-Bildgebung eines menschlichen oder tierischen Organismus nach Anspruch 1, wobei die Nanopartikel das Nanomaterial ZnGa₂₍₁₋ₓ₎Cr₂ₓO₄ mit x zwischen 0,001 und 0,0075 inklusive umfassen.

3. Verfahren der optischen in-vivo-Bildgebung eines menschlichen oder tierischen Organismus nach einem der Ansprüche 1-2, wobei die Nanopartikel das Nanomaterial ZnGa_{1.995}Cr_{0.005}O₄ umfassen.

4. Verfahren der optischen in-vivo-Bildgebung eines menschlichen oder tierischen Organismus nach einem der Ansprüche 1-3, wobei die Verabreichung der Nanopartikel zuvor auf dem intravenösen, intraarteriellen, intramuskulären, intraperitonealen oder retro-orbitalen Weg durchgeführt wird.

5. Verfahren der optischen in-vivo-Bildgebung eines menschlichen oder tierischen Organismus nach einem der Ansprüche 1-4, wobei die Nanopartikel eine Größe zwischen 1 und 10³ nm inklusive haben.

6. Verfahren der optischen in-vivo-Bildgebung eines menschlichen oder tierischen Organismus nach einem der Ansprüche 1-5, wobei die Nanopartikel auf der Oberfläche gepfropft sind, vorzugsweise mit einem Liganden, oder umhüllt.

7. Verfahren der optischen in-vivo-Bildgebung eines menschlichen oder tierischen Organismus nach einem der Ansprüche 1-6, wobei die Nanopartikel in mesoporöses Siliziumoxid gekapselt sind, was die Ladung und den Austrag von interessierenden Molekülen erlaubt.

8. Verfahren der biomodalen in-vivo-Bildgebung eines menschlichen oder tierischen Organismus, das die folgenden Schritte umfasst:
a) Erregung der persistenten Lumineszenz von Nanopartikeln durch in vivo-Bestrahlung in einer Wellenlänge zwischen 550 et 1000 nm inklusive des gesamten oder eines Teils des menschlichen oder tierischen Organismus, wobei die Nanopartikel dem Organismus zuvor verabreicht wurden und wobei die Nanopartikel nach einer Lichterregung auf Wellenlängen zwischen 550 und 1000 nm inklusive Photonen mit Wellenlängen zwischen 550 und 1000 nm inklusive während mindestens 0,01 Sekunde senden und wobei die Nanopartikel paramagnetische Eigenschaften haben und wobei die Nanopartikel ein Nanomaterial umfassen, das aus einer Matrix besteht, die aus den Gallaten, den Aluminaten, den Indaten, den Galliumoxiden, den Indiumoxiden, den Magnesiumoxiden, den Gallogermanaten, den Aluminogallaten, den Zink- und Galliumoxisulfiden, den Zink- und Galliumseleniden, den Zink- und Galliumoxitelluren ausgewählt ist, wobei die Matrix mit einem Übergangsmetall oder einem Lanthanid dotiert ist, das aus dem Chrom, dem Europium, dem Cerium, dem Nickel, dem Eisen, dem Kupfer und dem Kobalt ausgewählt ist und mit mindestens einem paramagnetischen Element, das aus Cr³⁺; Mn²⁺; Gd³⁺; Fe³⁺ und Ni³⁺ ausgewählt ist;
b) in vivo-Detektion der Nanopartikel im gesamten oder in einem Teil des menschlichen oder tierischen Organismus durch Messen der persistenten Lumineszenz der Nanopartikel durch optische Bildgebung; und
c) in vivo-Detektion der Nanopartikel im gesamten oder in einem Teil des menschlichen oder tierischen Organismus durch Magnetresonanzbildgebung.

9. Verfahren der biomodalen in-vivo-Bildgebung eines menschlichen oder tierischen Organismus nach Anspruch 8, wobei die Nanopartikel das Nanomaterial ZnGa_{2(1-x-y)}Cr₂ₓGd_{2y}O₄ mit x zwischen 0,001 und 0,0075 inklusive und dort zwischen 0,01 und 0,08 inklusive umfassen.

10. Verfahren der biomodalen in-vivo-Bildgebung eines menschlichen oder tierischen Organismus nach einem der Ansprüche 8-9, wobei die Nanopartikel das Nanomaterial ZnGa_{1.955}Cr_{0.005}Gd_{0.04}O₄ umfassen.

11. Verfahren der biomodalen in-vivo-Bildgebung eines menschlichen oder tierischen Organismus nach einem der Ansprüche 8-10, wobei die Verabreichung der Nanopartikel zuvor auf dem intravenösen, intraarteriellen, intramuskulären, intraperitonealen oder retro-orbitalen Weg durchgeführt wird.

12. Verfahren der biomodalen in-vivo-Bildgebung eines menschlichen oder tierischen Organismus nach einem der Ansprüche 8-11, wobei die Nanopartikel eine Größe zwischen 1 und 10³ nm inklusive haben.

13. Verfahren der biomodalen in-vivo-Bildgebung eines menschlichen oder tierischen Organismus nach einem der Ansprüche 8-12, wobei die Nanopartikel auf der Oberfläche vorzugsweise mit einem Liganden gepfropft sind oder umhüllt.

14. Verfahren der biomodalen in-vivo-Bildgebung eines menschlichen oder tierischen Organismus nach einem der Ansprüche 8-13, wobei die Nanopartikel in mesoporöses Siliziumoxid gekapselt sind, was die Ladung und den Austrag von interessierenden Molekülen erlaubt.

15. Nanopartikel, umfassend das Nanomaterial ZnGa_{2(1-x-y)}Cr₂ₓGd_{2y}O₄ mit x zwischen 0,001 und 0, 0075 inklusive und dort zwischen 0,01 und 0,08 inklusive, wobei das Nanomaterial insbesondere ZnGa_{1.955}Cr_{0.005}Gd_{0.04}O₄ ist.

16. Nanopartikel nach Anspruch 15, **dadurch gekennzeichnet, dass** seine Größe zwischen 1 und 10³ nm ist.

## Claims

1. An *in vivo* optical imaging method of a human or animal body, comprising the following steps:
a) exciting the persistent luminescence of nanoparticles by *in vivo* irradiation of all or part of the human or animal body at a wavelength between 550 and 1000 nm, said nanoparticles being previously administered to the human or animal body, and said nanoparticles emitting photons at wavelengths between 550 and 1000 nm for at least 0.01 second, after light excitation at wavelengths between 550 and 1000 nm, and said nanoparticles comprising a nanomaterial formed of a matrix selected from among gallates, aluminates, indates, and their mixed compounds gallo-germanates, gallo-aluminates, gallo-indates, gallium oxides, indium oxides, magnesium oxides, zinc and gallium oxysulfides, zinc and gallium oxyselenides, zinc and gallium oxytellurides, said matrix being doped with a transition metal or lanthanide selected from among chromium, europium, cerium, nickel, iron, copper and cobalt; and
b) detecting the nanoparticles *in vivo* in all or part of the human or animal body by measuring the persistent luminescence of the nanoparticles via optical imaging.

2. The *in vivo* optical imaging method of a human or animal body according to claim 1, wherein said nanoparticles comprise the nanomaterial ZnGa₂₍₁₋ₓ₎Cr₂ₓO₄ with x between 0.001 and 0.0075.

3. The *in vivo* optical imaging method of a human or animal body according to one of claims 1-2, wherein said nanoparticles comprise the nanomaterial ZnGa_{1.995}Cr_{0.005}O₄.

4. The *in vivo* optical imaging method of a human or animal body according to one of claims 1-3, wherein the administering of the nanoparticles is previously performed via intravenous, intra-arterial, intramuscular, intraperitoneal or retro-orbital route.

5. The *in vivo* optical imaging method of a human or animal body according to one of claims 1-4, wherein the size of the nanoparticles is between 1 and 10³ nm.

6. The *in vivo* optical imaging method of a human or animal body according to one of claims 1-5, wherein the nanoparticles are surface grafted, advantageously with a ligand, or coated.

7. The *in vivo* optical imaging method of a human or animal body according to one of claims 1-6, wherein the nanoparticles are encapsulated in mesoporous silica allowing the loading and release of molecules of interest.

8. A bimodal *in vivo* imaging method of a human or animal body comprising the following steps:
a) exciting the persistent luminescence of the nanoparticles by *in vivo* irradiation of all or part of the human or animal body at a wavelength between 550 and 1000 nm, said nanoparticles being previously administered to the human or animal body, and said nanoparticles emitting photons at wavelengths between 550-1000 nm for at least 0.01 second under excitation light at wavelengths between 550 and 1000 nm, said nanoparticles having paramagnetic properties and said nanoparticles comprising a nanomaterial formed of a matrix from among gallates, aluminates, indates, gallium oxides, indium oxides, magnesium oxides, gallo-germanates, alumina-gallates, zinc and gallium oxysulfides, zinc an gallium oxyselenides, zinc and gallium oxytellurides, said matrix being doped with a transition metal or lanthanide selected from among chromium, europium, cerium, nickel, iron, copper and cobalt and with at least one paramagnetic element selected from among Cr³⁺; Mn²⁺; Gd³⁺; Fe³⁺ and Ni³⁺;
b) detecting the nanoparticles *in vivo* in all or part of the human or animal body by measuring the persistent luminescence of the nanoparticles using optical imaging; and
c) detecting the nanoparticles *in vivo* in all or part of the animal body by magnetic resonance imaging.

9. The *in vivo* bimodal imaging method of a human or animal body according to claim 8 wherein said nanoparticles comprise the nanomaterial ZnGa_{2(1-x-y)}Cr₂ₓGd_{2y}O₄ with x between 0.001 and 0.0075 and y between 0.01 and 0.08.

10. The *in vivo* bimodal imaging method of a human or animal body according to one of claims 8-9, wherein said nanoparticles comprise the nanomaterial ZnGa_{1.955}Cr_{0.005}Gd_{0.04}O₄.

11. The *in vivo* bimodal imaging method of a human or animal body according to one of claims 8-10, wherein the administering of the nanoparticles is previously performed via intravenous, intra-arterial, intramuscular, intraperitoneal or retro-orbital route.

12. The *in vivo* bimodal imaging method of a human or animal body according to one of claims 8-11, wherein the size of the nanoparticles is between 1 and 10³ nm.

13. The *in vivo* bimodal imaging method of a human or animal body according to one of claims 8-12, wherein the nanoparticles are surface grafted, advantageously with a ligand, or coated.

14. The *in vivo* bimodal imaging method of a human or animal body according to one of claims 8-13, wherein the nanoparticles are encapsulated in mesoporous silica allowing the loading and release of molecules of interest.

15. A nanoparticle comprising the nanomaterial ZnGa_{2(1-x-y)}Cr₂ₓGd_{2y}O₄ with x between 0.001 and 0.0075 and y between 0.01 and 0.08, in particular the nanomaterial is ZnGa_{1.955}Cr_{0.005}Gd_{0.04}O₄.

16. The nanoparticle according to claim 15, **characterized in that** its size is between 1 and 10³ nm.
